# EUROPEAN PATENT APPLICATION

(11) **EP 3 640 339 A1**
(43) Date of publication of application: **22.04.2020**
(21) Application number: 18817351.2
(22) Date of filing: 15.06.2018
(51) Int. Cl.: C12Q 1/02, G01N 33/15

(54) **NOVEL SCREENING METHOD FOR TGF- INHIBITOR**

(30) Priority: 16.06.2017 JP 2017118615
(71) Applicant: The Doshisha, Kyoto-shi, Kyoto 602-8580 (JP)
(72) Inventor: KOIZUMI Noriko, Kyotanabe-shi Kyoto 610-0394 (JP); OKUMURA Naoki, Kyotanabe-shi Kyoto 610-0394 (JP)
(74) Representative: J A Kemp LLP
(86) International application number: PCT/JP2018/022944
(87) International publication number: WO 2018/230712

(57) **Abstract**

The present invention provides a method for screening for a compound that is effective against disorders of the corneal endothelium. The method according to the present invention comprises: (a) a step for bringing candidate compounds into contact with immortalized cells of Fuchs' corneal endothelial dystrophy in the presence of transforming growth factor (TGF)-β, making evaluations on the inhibitory activity of said candidate compounds against said TGF-β, and selecting a compound having the inhibitory activity; (b) a step for evaluating the toxicity of the compound selected in step (a) with respect to said immortalized cells; (c) a step for evaluating the inhibitory activity of the compound selected in step (a) against said TGF-β; and (d) a step for selecting a compound that has been evaluated to have low toxicity to said immortalized cells in step (b) and has been evaluated to have inhibitory activity against said TGF-β in step (c). The method according to the present invention makes it possible to screen for a compound that is effective for disorders of the corneal endothelium.

## Description

### [Technical Field]

The present invention relates to a method, kit, and the like for screening a compound effective against a corneal endothelial disease using a Fuchs' endothelial corneal dystrophy model system.

### [Background Art]

Visual information is recognized when light transmitted into the cornea, which is a transparent tissue at the front-most part of an eye ball, reaches the retina and excites nerve cells of the retina, and a generated electric signal is transmitted through the optic nerve to the visual cortex of the cerebrum. To attain good vision, it is necessary that the cornea is transparent. The transparency of the cornea is retained by maintaining constant water content with pumping and barrier functions of corneal endothelial cells.

Human corneal endothelial cells are present at a density of about 3000 cells per 1 mm² at birth. Once damaged, human corneal endothelial cells do not have the ability to regenerate. Fuchs' endothelial corneal dystrophy is a disease causing abnormality in endothelial cells inside the cornea, resulting in edema of the cornea. The details of the cause thereof is unknown. In Fuchs' endothelial corneal dystrophy, extracellular matrix such as collagen is deposited on a part of the back surface of a Descemet's membrane at the back of the cornea, forming guttae (Corneal guttae) and resulting in hypertrophy of the Descemet's membrane, which are the cause of photophobia or blurred vision. It is understood that there is no effective therapeutic method other than corneal transplant for Fuchs' endothelial corneal dystrophy. However, there is a shortage in cornea donation in Japan, where the number of patients waiting for corneal transplant is about 2600, whereas the number of corneal transplants performed in Japan is approximately 1700 annually.

Although animals were mainly used in the past for test research regarding safety and effectiveness of pharmaceutical products, research at the implementation level are being carried out regarding techniques for in vitro test researches of effectiveness and safety of a pharmaceutical product using cultured cells or the like instead of using animals from the viewpoint of animal protection. For example, animal tests are being carried out after a pre-test with a method using primary cultured cells obtained from a tissue of a living organism or immortalized cell (established cell) system that infinitely grows. However, although primary cells grow well at an initial stage, the growth will gradually stop (cell aging) with subculturing, or exhibit transformation. Furthermore, the primary cells have been pointed out to change with passaging in addition to the problem of inhomogeneity wherein the characteristics thereof differ every time the cells are collected from a tissue of a living organism. Specifically, when the growth speed is very slow or when the cells are derived from a micro organ, it is very difficult to obtain primary cells that are enough to be provided for a test.

Patent Literature 1 discloses a method for producing immortalized Fuchs' endothelial corneal dystrophy cells. Patent Literature 2 uses immortalized Fuchs' endothelial corneal dystrophy cells for confirming cell injury inhibitory effect. However, there has been no report of a quick and easy screening method with high reliability for screening an effective and highly safe compound in a corneal endothelial disease.

### [Citation List]

### [Patent Literature]

[PTL 1] International Publication No. WO 2015/015655
[PTL 2] International Publication No. WO 2015/015654

### [Summary of Invention]

### [Solution to Problem]

The inventors have found that it is possible to select a compound that is effective against a corneal endothelial disease by using TGF-β inhibiting activity upon contacting a candidate compound with immortalized Fuchs' endothelial corneal dystrophy cells in the presence of TGF-β as an indicator and also achieved selection of a compound with higher safety by carrying out toxicity evaluation. Furthermore, selection of a candidate compound with higher reliability became possible by processing and ranking the score of TGF-β inhibiting activity and the score of toxicity. The inventors also found a method of carrying out such a screening in high throughput.

The present invention therefore provides the following inventions.
(Item 1)
   A method for screening a compound effective against a corneal endothelial condition, disorder, or disease, the method comprising:
   (a) a step of contacting a candidate compound with immortalized Fuchs' endothelial corneal dystrophy cells in the presence of a transforming growth factor (TGF)-β to evaluate inhibiting activity of the candidate compound against the TGF-β and selecting a compound having the inhibiting activity;
   (b) a step of evaluating toxicity of the compound selected in the step (a) to the cells;
   (c) a step of evaluating the inhibiting activity of the compound selected in the step (a) against the TGF-β, if necessary; and
   (d) a step of selecting a compound evaluated to be less toxic to the cells in the step (b) and evaluated to have the inhibiting activity against the TGF-β in the step (a) or the step (c).
(Item 2)
   The method of item 1, wherein the inhibiting activity against the TGF-β is activity of inhibiting apoptosis of the cells due to the TGF-β signal.
(Item 3)
   The method of item 1 or 2, wherein the steps (a) and (c) comprise measuring caspase activity, wherein the inhibiting activity against the TGF-β is caspase activity which is substantially decreased compared to a candidate compound non-contacted group.
(Item 4)
   The method of any one of items 1 to 3, wherein the number of procedures of evaluation of the inhibiting activity against the TGF-β in the step (c) is larger than the number of procedures of evaluation of the inhibiting activity against the TGF-β in the step (a).
(Item 5)
   The method of any one of items 1 to 4, wherein the number of procedures of evaluation of the inhibiting activity against the TGF-β in the step (c) is n = 4 to 6 and the number of procedures of evaluation of the inhibiting activity against the TGF-β in the step (a) is n = 1 to 3.
(Item 6)
   The method of any one of items 3 to 5, wherein the caspase activity is Caspase 3/7 activity.
(Item 7)
   The method of any one of items 1 to 6, wherein a compound exhibiting Caspase 3/7 activity that is about 75% or less compared to a candidate compound non-contacted group is selected as a compound having the inhibiting activity against the TGF-β in the step (a).
(Item 8)
   The method of any one of items 1 to 7, wherein the step (b) comprises measuring cell viability, wherein the toxicity is evaluated to be low when the cell viability is not substantially reduced.
(Item 9)
   The method of any one of items 1 to 8, wherein a compound exhibiting cell viability which is not substantially reduced and Caspase 3/7 activity which is substantially decreased compared to a candidate compound non-contacted group is selected in the step (d).
(Item 10)
   The method of any one of items 1 to 9, wherein a compound exhibiting about 90% or more of the cell viability and exhibiting a ratio of Caspase 3/7 activity (%) to the cell viability (%) that is about 0.8 or less is selected in the step (d).
(Item 11)
   The method of any one of items 1 to 10, wherein the corneal endothelial condition, disorder, or disease is Fuchs' endothelial corneal dystrophy.
(Item 12)
   The method of any one of items 1 to 11, further comprising:
   (e) a step of contacting a compound selected in the step (d) with immortalized human corneal endothelial cells in the presence of an endoplasmic reticulum associated stress inducing substance to evaluate inhibiting activity of a compound selected in the step (d) against the endoplasmic reticulum associated stress inducing substance;
   (f) a step of evaluating toxicity of a compound selected in the step (d) against the immortalized human corneal endothelial cells; and
   (g) a step of selecting a compound which was evaluated to be less toxic to the immortalized human corneal endothelial cells in step (f) and evaluated to have the inhibiting activity against the endoplasmic reticulum associated stress inducing substance in step (e).
(Item 13)
   The method of item 12, wherein the inhibiting activity against the endoplasmic reticulum associated stress inducing substance is activity of inhibiting apoptosis of the immortalized human corneal endothelial cells due to the endoplasmic reticulum associated stress inducing substance.
(Item 14)
   The method of item 12 or 13, wherein the step (e) comprises measuring caspase activity, wherein the inhibiting activity against the endoplasmic reticulum associated stress inducing substance is caspase activity which is substantially decreased compared to a candidate compound non-contacted group.
(Item 15)
   The method of item 14, wherein the caspase activity is Caspase activity 3/7.
(Item 16)
   The method of any one of items 12 to 15, wherein a compound exhibiting Caspase 3/7 activity that is about 75% or less compared to a candidate compound non-contacted group is selected as a compound having the inhibiting activity against the endoplasmic reticulum associated stress inducing substance in the step (g).
(Item 17)
   The method of any one of items 12 to 16, wherein the step (f) comprises measuring cell viability, wherein the toxicity is evaluated to be low when the cell viability is not substantially reduced.
(Item 18)
   The method of any one of items 12 to 17, wherein a compound exhibiting cell viability which is not substantially reduced and Caspase 3/7 activity which is substantially decreased compared to a candidate compound non-contacted group is selected in the step (g).
(Item 19)
   The method of any one of items 12 to 18, wherein a compound exhibiting about 90% or more of the cell viability and exhibiting a ratio of Caspase 3/7 activity to the cell viability (%) that is about 0.8 or less is selected in in the step (g).
(Item 20)
   The method of any one of items 12 to 19, wherein the corneal endothelial condition, disorder, or disease is Fuchs' endothelial corneal dystrophy.
(Item 21)
   The method of any one of items 12 to 20, wherein the endoplasmic reticulum associated stress inducing substance is selected from the group consisting of MG-132, thapsigargin, and tunicamycin.
(Item 22)
   The method of any one of items 12 to 21, wherein the endoplasmic reticulum associated stress inducing substance is MG-132.
(Item 23)
   A method for screening a compound effective against a corneal endothelial condition, disorder, or disease, the method comprising:
   (a) a step of contacting a candidate compound with immortalized Fuchs' endothelial corneal dystrophy cells in the presence of a transforming growth factor (TGF)-β to evaluate caspase activity of the candidate compound and selecting a compound with substantially decreased caspase activity compared to a candidate compound non-contacted group;
   (b) a step of evaluating toxicity of the compound selected in the step (a) to the immortalized Fuchs' endothelial corneal dystrophy cells;
   (c) a step of evaluating the caspase activity of the compound selected in the step (a);
   (d) a step of selecting compounds evaluated to be less toxic to the immortalized Fuchs' endothelial corneal dystrophy cells in the step (b) and evaluated to have caspase activity which is substantially decreased in the step (c);
   (e) a step of contacting a compound selected in the step (d) with immortalized human corneal endothelial cells in the presence of MG-132 to evaluate caspase activity of a candidate compound in compounds selected in the step (d);
   (f) a step of evaluating toxicity of the immortalized human corneal endothelial cells of a compound selected in the step (e); and
   (g) a step of selecting a compound which was evaluated to be less toxic to the immortalized human corneal endothelial cells in the step (f) and evaluated to have caspase activity which is substantially decreased in the step (e).
(Item 24)
   A kit for screening a compound effective against a corneal endothelial condition, disorder, or disease using the method of any one of items 1 to 11, the kit comprising:
   a TGF-β;
   immortalized Fuchs' endothelial corneal dystrophy cells;
   a reagent or device for measuring the inhibiting activity against the TGF-β; and
   a reagent or device for measuring cell toxicity.
(Item 25)
   The kit of item 24, wherein the TGF-β is TGF-β2.
(Item 26)
   The kit of item 24 or 25, wherein the immortalized Fuchs' endothelial corneal dystrophy cells are from corneal endothelial cells from a Fuchs' endothelial corneal dystrophy patient, to which an SV40 large T antigen and an hTERT gene were introduced.
(Item 27)
   The kit of any one of items 24 to 26 wherein the immortalized Fuchs' endothelial corneal dystrophy cells are from immortalized cells from a Fuchs' endothelial corneal dystrophy patient which were cultured in a medium comprising 4-[4-(1,3-benzodioxole-5-yl)-5-(2-pyridinyl)-1H-imidazole-2-yl]benzamide and 4-[4-(4-fluorophenyl)-2-(4-methylsulfinylphenyl)-1H-imidazole-5-yl]pyridine).
(Item 28)
   The kit of items 24 to 27, wherein the reagent or device for measuring the inhibiting activity against the TGF-β is a reagent or device for measuring caspase activity.
(Item 29)
   The kit of any one of items 24to 28, wherein the caspase activity is Caspase 3/7 activity.
(Item 30)
   The kit of any one of items 24 to 29, wherein the reagent or device for measuring the cell toxicity is a reagent or device for measuring cell viability.
(Item 31)
   The kit of any one of items 24 to 30, wherein the reagent or device for measuring the inhibiting activity against the TGF-β comprises a luminescence reagent.
(Item 32)
   The kit of any one of items 24 to 31, wherein the reagent or device for measuring the cell toxicity comprises a luminescence reagent.
(Item 33)
   A method for screening a compound effective against a corneal endothelial condition, disorder, or disease, the method comprising:
   (a) a step of contacting a candidate compound with immortalized human corneal endothelial cells in the presence of an endoplasmic reticulum associated stress inducing substance to evaluate inhibiting activity of the candidate compound against the endoplasmic reticulum associated stress inducing substance and selecting a compound having the inhibiting activity;
   (b) a step of evaluating toxicity of the compound selected in the step (a) to the cells;
   (c) a step of evaluating the inhibiting activity of the compound selected in the step (a) against the endoplasmic reticulum associated stress inducing substance, if necessary; and
   (d) a step of selecting a compound evaluated to be less toxic to the cells in the step (b) and evaluated to have the inhibiting activity against the endoplasmic reticulum associated stress inducing substance in the step (a) or the step (c).
(Item 34)
   The method of item 33, wherein the inhibiting activity against the endoplasmic reticulum associated stress inducing substance is activity of inhibiting apoptosis of the cells due to the endoplasmic reticulum associated stress inducing substance.
(Item 35)
   The method of items 33 or 34, wherein the steps (a) and (c) comprise measuring caspase activity, wherein the inhibiting activity against the endoplasmic reticulum associated stress inducing substance is caspase activity which is substantially decreased compared to a candidate compound non-contacted group.
(Item 36)
   The method of any one of items 33 to 35, wherein the number of procedures of evaluation of the inhibiting activity against the endoplasmic reticulum associated stress inducing substance in the step (c) is larger than the number of procedures of evaluation of the inhibiting activity against the endoplasmic reticulum associated stress inducing substance in the step (a).
(Item 37)
   The method of any one of items 33 to 36, wherein the number of procedures of evaluation of the inhibiting activity against the endoplasmic reticulum associated stress inducing substance in the step (c) is n = 4 to 6 and the number of procedures of evaluation of the inhibiting activity against the endoplasmic reticulum associated stress inducing substance in the step (a) is n = 1 to 3.
(Item 38)
   The method of any one of items 33 to 37, wherein the caspase activity is Caspase 3/7 activity.
(Item 39)
   The method of any one of items 33 to 38, wherein a compound exhibiting Caspase 3/7 activity that is about 75% or less compared to a candidate compound non-contacted group is selected as a compound having the inhibiting activity against the endoplasmic reticulum associated stress inducing substance in the step (a).
(Item 40)
   The method of any one of items 33 to 39, wherein the step (b) comprises measuring cell viability, wherein the toxicity is evaluated to be low when the cell viability is not substantially reduced.
(Item 41)
   The method of any one of items 33 to 40, wherein a compound exhibiting cell viability which is not substantially reduced and Caspase 3/7 activity which is substantially decreased compared to a candidate compound non-contacted group is selected in the step (d).
(Item 42)
   The method of any one of items 33 to 41, wherein a compound exhibiting 90% or more of the cell viability and exhibiting a ratio of Caspase 3/7 activity (%) to the cell viability (%) that is about 0.8 or less is selected in the step (d).
(Item 43)
   The method of any one of items 33 to 42, wherein the corneal endothelial condition, disorder, or disease is Fuchs' endothelial corneal dystrophy.
(Item 44)
   The method of any one of items 33 to 43, wherein the endoplasmic reticulum associated stress inducing substance is selected from the group consisting of MG-132, thapsigargin, and tunicamycin.
(Item 45)
   The method any one of items 33 to 44, wherein the endoplasmic reticulum associated stress inducing substance is MG-132.
(Item 46)
   A kit for screening a compound effective against a corneal endothelial condition, disorder, or disease using the method of items 33 to 45, the kit comprising:
   an endoplasmic reticulum associated stress inducing substance;
   immortalized human corneal endothelial cells;
   a reagent or device for measuring the inhibiting activity against the endoplasmic reticulum associated stress inducing substance; and
   a reagent or device for measuring cell toxicity.
(Item 47)
   The kit of item 46, wherein the immortalized human corneal endothelial cells are from normal corneal endothelial cells, to which an SV40 large T antigen and an hTERT gene were introduced.
(Item 48)
   The kit of item 46 or 47 wherein the immortalized human corneal endothelial cells are from normal immortalized cells which were cultured in a medium comprising 4-[4-(1,3-benzodioxole-5-yl)-5-(2-pyridinyl)-1H-imidazole-2-yl]benzamide and 4-[4-(4-fluorophenyl)-2-(4-methylsulfinylphenyl)-1H-imidazole-5-yl]pyridine).
(Item 49)
   The kit of any one of items 46 to 48, wherein the reagent for measuring the inhibiting activity against the TGF-β is a reagent for measuring caspase activity.
(Item 50)
   The kit of any one of items 46 to 49, wherein the caspase activity is Caspase 3/7 activity.
(Item 51)
   The kit of items 46 to 50, wherein the reagent for measuring the cell toxicity is a reagent for measuring cell viability.
(Item 52)
   The kit of any one of items 46 to 51, wherein the endoplasmic reticulum associated stress inducing substance is selected from the group consisting of MG-132, thapsigargin, and tunicamycin.
(Item 53)
   The kit of any one of items 46 to 52, wherein the endoplasmic reticulum associated stress inducing substance is MG-132.
(Item 54)
   The kit of any one of items 46 to 53, wherein the reagent or device for measuring the inhibiting activity against the endoplasmic reticulum associated stress inducing substance comprises a luminescence reagent.
(Item 55)
   The kit of any one of items 46 to 54, wherein the reagent or device for measuring the cell toxicity comprises a luminescence reagent.

The present invention is intended so that one or more of the aforementioned features can be provided not only as the explicitly disclosed combinations, but also as other combinations thereof. Additional embodiments and advantages of the present invention are recognized by those skilled in the art by reading and understanding the following detailed description, as needed.

### [Advantageous Effects of Invention]

The present invention provides a model system of Fuchs' endothelial corneal dystrophy and provides a technique available to industries (cell culturing industry, pharmaceutical, or the like) related to techniques such as screening for drugs to treat or prevent Fuchs' endothelial corneal dystrophy.

### [Brief Description of Drawings]

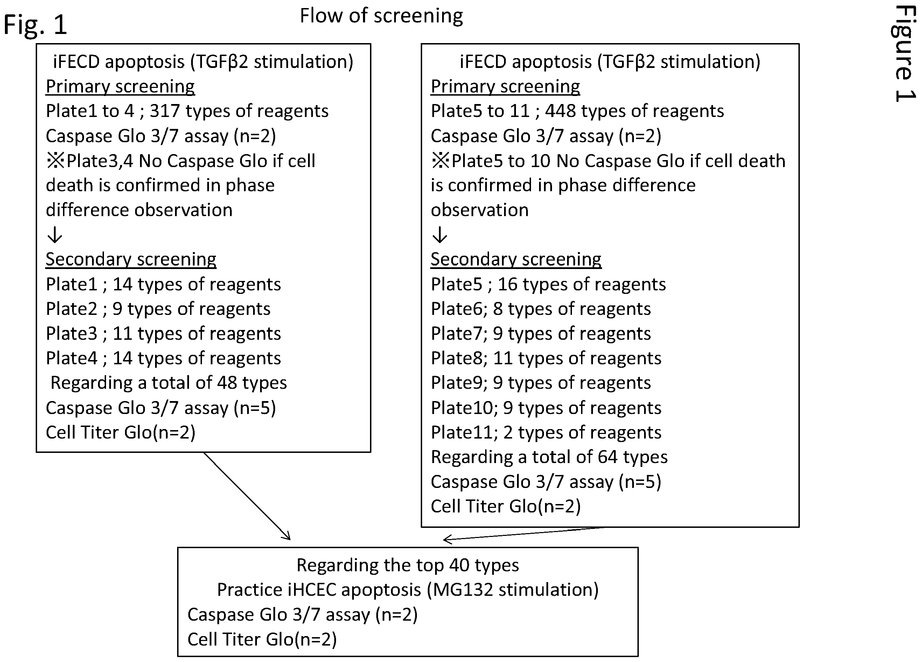

[Figure 1] Figure **1** is a typical schematic diagram of a screening method using immortalized Fuchs' endothelial corneal dystrophy cells (iFECD) and a screening method using immortalized human corneal endothelial cells (iHCEC).

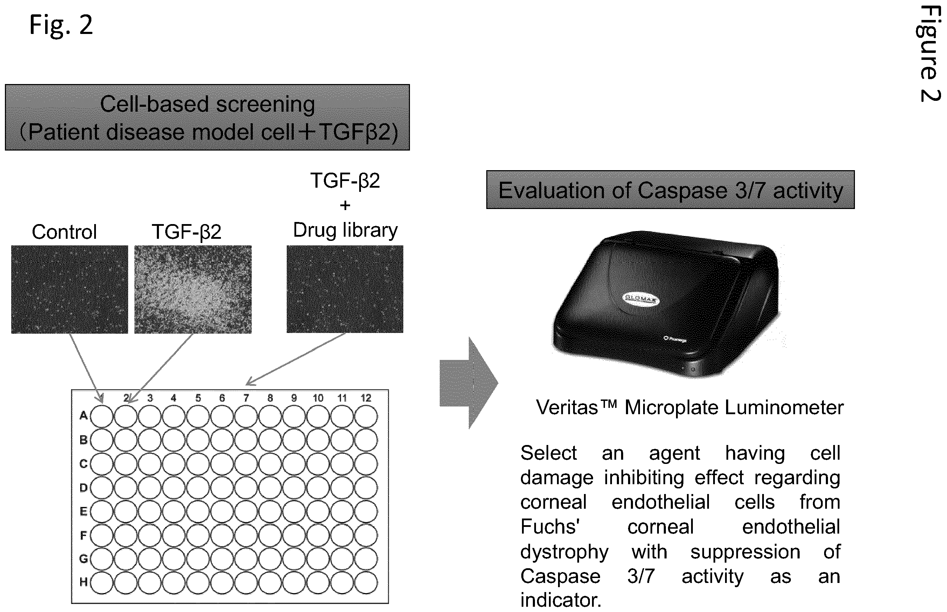

[Figure 2] Figure **2** shows an outline of screening using immortalized Fuchs' endothelial corneal dystrophy cells (iFECD).

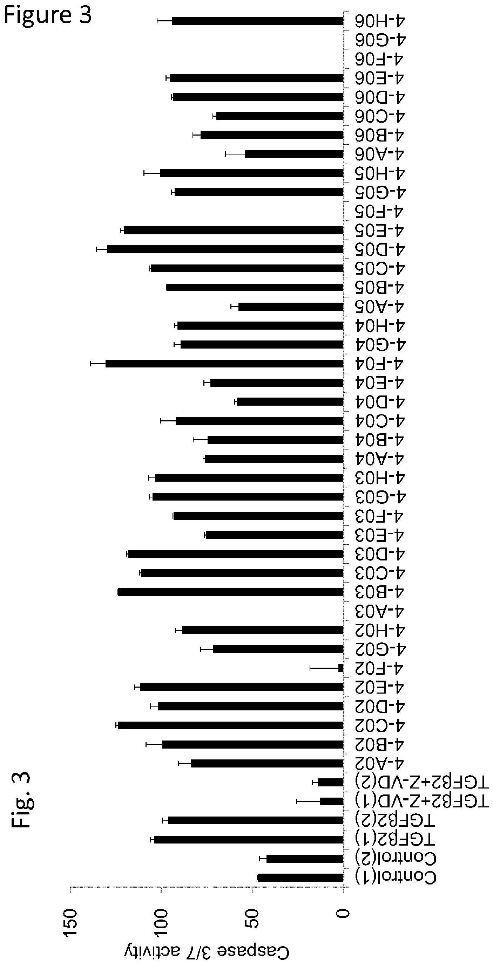

[Figure 3] Figure **3** shows typical results of Caspase 3/7 activity measured by Caspase-Glo® 3/7 Assay in a primary screening. The vertical axis shows caspase activity (%) vs a candidate compound non-contacted group (TGF-β supplemented group). The horizontal axis shows, from the left, plate positions of control group, TGF-β2(5ng/mL) supplemented group, TGF-β2(5ng/mL) + Z-VD-FMK supplemented group (10 µM) and TGF-β2 (5 ng/mL) + each agent of SCREEN-WELL® FDA Approved Drug Library V2, Japan version (10µM) supplemented group.

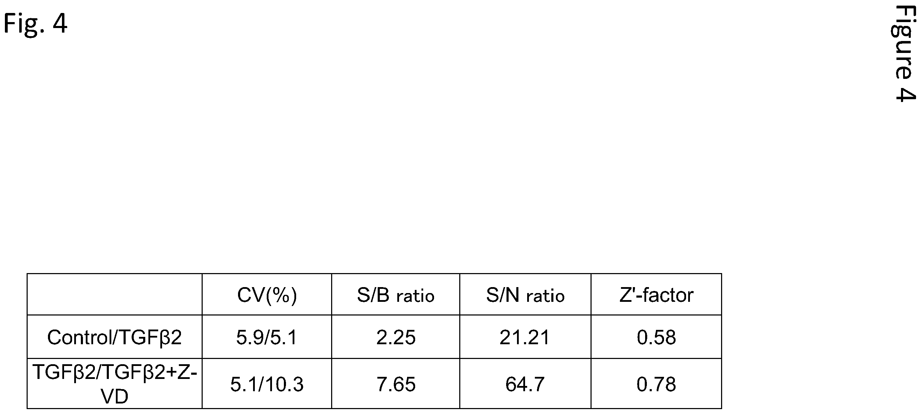

[Figure 4] Figure **4** shows results of calculating indicators (CV value, S/B ratio, S/N ratio and Z'-factor) of when the control is set as 0% and the post-TGF-β2 reaction is set as 100% and when the post-TGF-β2 reaction is set as 100% and the positive control post-TGF-β2 + Z-VD-FMK reaction is set as 0%.

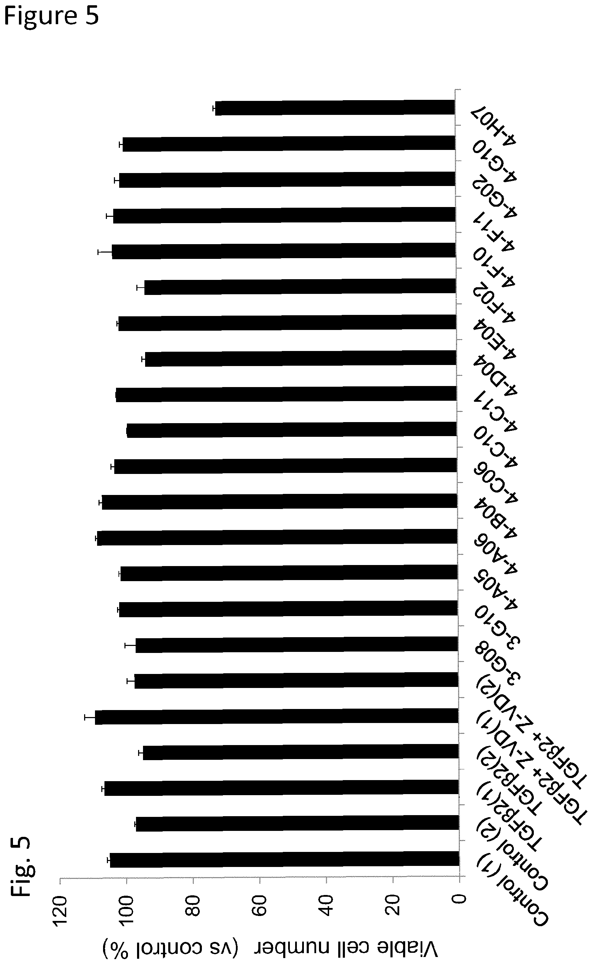

[Figure 5] Figure **5** shows typical results of measurement of cell viability in a secondary screening. The vertical axis shows cell viability (%) vs a candidate compound non-contacted group. The horizontal axis shows, from the left, plate positions of control group, TGF-β2(5ng/mL) supplemented group, TGF-β2(5ng/mL) + Z-VD-FMK supplemented group (10 µM) and TGF-β2 (5 ng/mL) + each agent of SCREEN-WELL® FDA Approved Drug Library V2, Japan version (10µM) supplemented group.

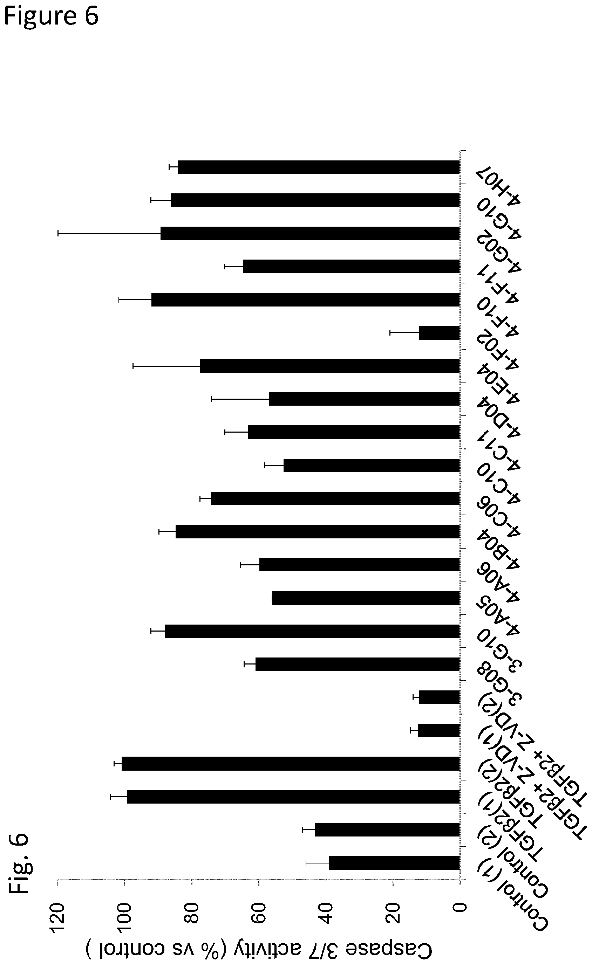

[Figure 6] Figure **6** shows typical results of measurement of Caspase 3/7 activity in a secondary screening. The vertical axis shows Caspase 3/7 activity (%) vs a candidate compound non-contacted group. The horizontal axis shows, from the left, plate positions of control group, TGF-β2(5ng/mL) supplemented group, TGF-β2(5ng/mL) + Z-VD-FMK supplemented group (10 µM) and TGF-β2 (5 ng/mL) + each agent of SCREEN-WELL® FDA Approved Drug Library V2, Japan version (10µM) supplemented group.

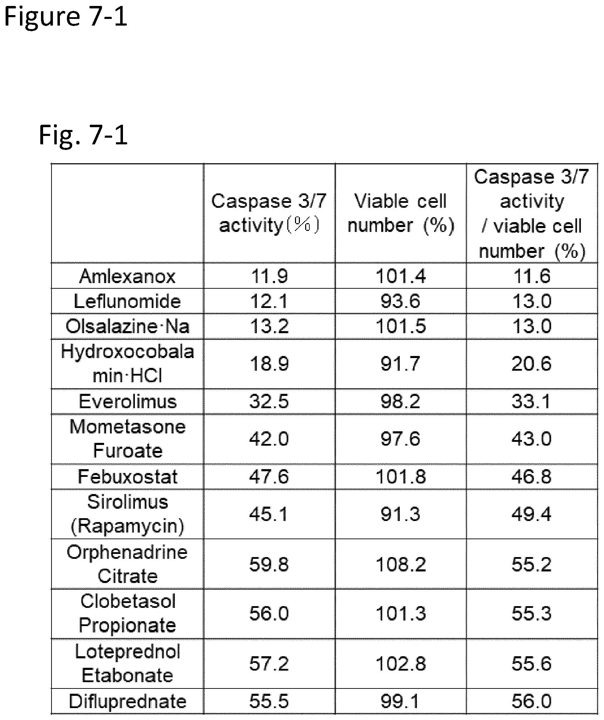

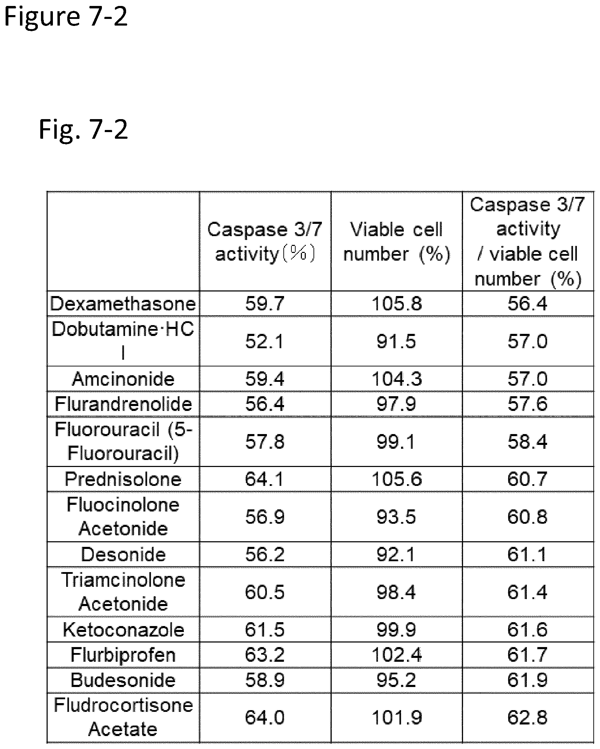

[Figure 7-1] Figure **7** shows the agent name of each agent of SCREEN-WELL® FDA Approved Drug Library V2, Japan version, results of the secondary screening of Caspase 3/7 activity under TGF-β2 stimulation, results of cell viability, and results of secondary screening of TGF-β2 stimulation. Figure **7** shows the agents of which value of Caspase 3/7 activity/cell viability (%) was ranked from the 1st to the 25th.
[Figure 7-2] Figure **7** shows the agent name of each agent of SCREEN-WELL® FDA Approved Drug Library V2, Japan version, results of the secondary screening of Caspase 3/7 activity under TGF-β2 stimulation, results of cell viability, and results of secondary screening of TGF-β2 stimulation. Figure **7** shows the agents of which the value of Caspase 3/7 activity/cell viability (%) was ranked from the 1st to the 25th.

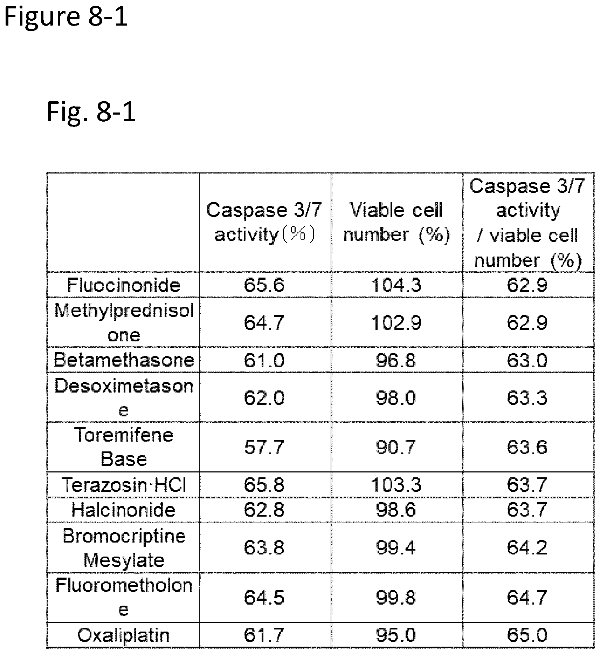

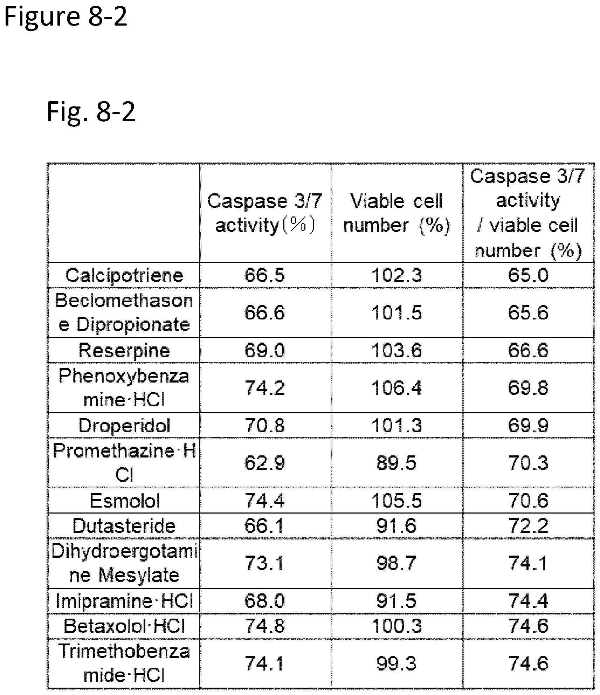

[Figure 8-1] Figure **8** shows the agent name of each agent of SCREEN-WELL® FDA Approved Drug Library V2, Japan version, results of the secondary screening of Caspase 3/7 activity under TGF-β2 stimulation, results of cell viability, and results of secondary screening of TGF-β2 stimulation. Figure **8** shows the agents of which the value of Caspase 3/7 activity/cell viability (%) was ranked from the 26th to the 47th.
[Figure 8-2] Figure **8** shows the agent name of each agent of SCREEN-WELL® FDA Approved Drug Library V2, Japan version, results of the secondary screening of Caspase 3/7 activity under TGF-β2 stimulation, results of cell viability, and results of secondary screening of TGF-β2 stimulation. Figure **8** shows the agents of which the value of Caspase 3/7 activity/cell viability (%) was ranked from the 26th to the 47th.

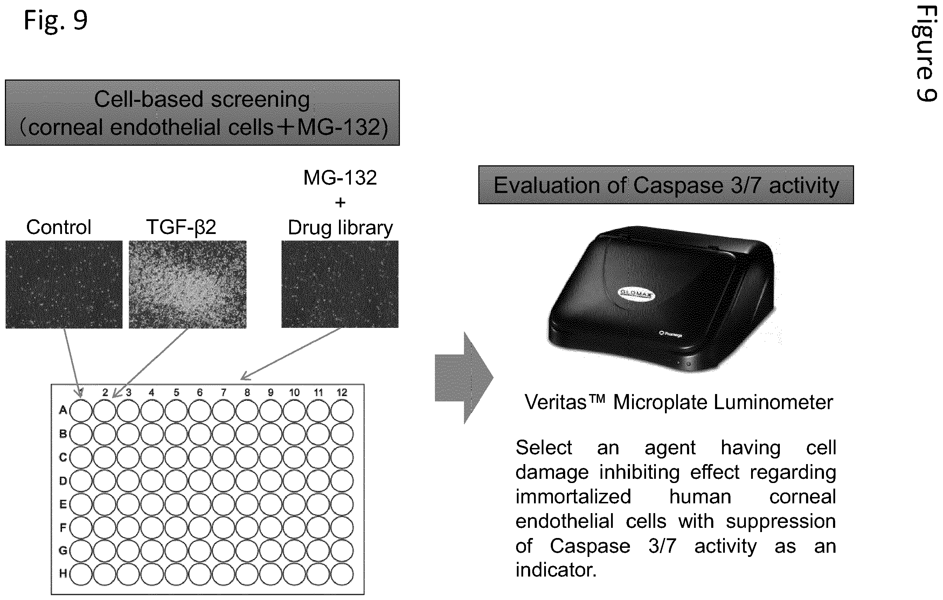

[Figure 9] Figure **9** shows an outline of screening using immortalized human corneal endothelial cells (iHCEC)

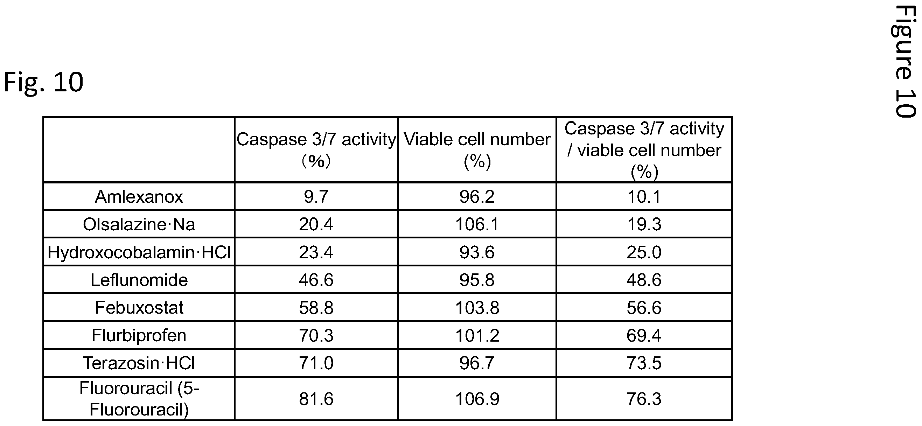

[Figure 10] Figure **10** shows the agent names, results of the screening of Caspase 3/7 activity in MG-132 (0.1 µM) stimulation, results of cell viability with MG-132 (0.1 µM) stimulation, and values obtained by dividing the result of screening in MG-132 (0.1 µM) stimulation by the cell viability ((Caspase 3/7 activity)/(cell viability) (%)).

### [Description of Embodiments]

The present invention is explained hereinafter. Throughout the entire specification, a singular expression should be understood as encompassing the concept thereof in the plural form, unless specifically noted otherwise. Thus, singular articles (e.g., "a", "an", "the", and the like in the case of English) should also be understood as encompassing the concept thereof in the plural form, unless specifically noted otherwise. Further, the terms used herein should be understood as being used in the meaning that is commonly used in the art, unless specifically noted otherwise. Therefore, unless defined otherwise, all terminologies and scientific technical terms that are used herein have the same meaning as the general understanding of those skilled in the art to which the present invention pertains. In case of a contradiction, the present specification (including the definitions) takes precedence.

### (Definition)

As used herein, "about" before a numerical value means ±10% of the numerical value that follows.

As used herein, "ocular cell" is used in a broad sense, referring to any cell that is present in an eye and encompassing any cell that is present in an eyelid, sclera, cornea, uvea, lens, vitreum, retina, or optic nerve.

As used herein, "corneal endothelial cell" is used in the meaning that is commonly used in the art. A cornea is one of the layer-like tissues constituting an eye that is transparent and is positioned at a portion that is closest to the outer environment. A cornea is understood to be made of five layers arranged in order from the outer side (body surface) in a human. A cornea is made up of corneal epithelium, Bowman's membrane (outer borderline), lamina propria, Descemet's membrane (inner borderline) and corneal endothelium, from the outer side. As referred to herein, the portions other than the epithelium and endothelium are collectively referred to as "corneal stroma", unless specified otherwise.

As used herein, "iFECD" (immortalized Fuchs' endothelial corneal dystrophy) is an abbreviation for immortalized Fuchs' endothelial corneal dystrophy cells. A manufacturing method of iFECD is described in detail in Patent Literature 1 and the entire content thereof is incorporated herein by reference.

As used herein, "HCEC" (human corneal endothelial cells) is an abbreviation for human corneal endothelial cells. "iHCEC" is an abbreviation for immortalized human corneal endothelial cells.

As used herein, "corneal tissue" is used in the meaning that is commonly used and refer to the tissue itself constituting a cornea. A corneal tissue may include all of (this is in case of a human; when in case of other animals, the word refers to the entirety thereof in accordance with the corresponding section) corneal epithelium, Bowman's membrane, lamina propria, Descemet's membrane and corneal endothelium, constituting a cornea, or a part of the above may be absent. Alternatively, the corneal tissue may include other tissues (sclera) of the cornea, in which case the corneal tissue may specifically be referred to as a sclerocornea.

As used herein, "undifferentiated cell" refers to any cell with differentiation ability. Therefore, other than a stem cell, an undifferentiated cell encompasses a cell that has differentiated to a certain degree but may further differentiate.

As used herein, "immortalized cell" refers to a cell line (strain) exhibiting a life span that appears to be infinite during culturing (having the ability to grow through infinite number of divisions) as the definitive feature thereof herein. Such an immortalized cell can escape cell aging and acquire the ability to infinitely grow while repeating the passage of the primary culture, or can give immortalization ability by introducing a specific gene to a cell of interest.

As used herein, "established" cell refers to a state wherein a specific property (e.g., pluripotency) is maintained and a cell continues to stably grow under a culturing condition. It is common that an immortalized cell often comprises a desired feature, thereby often corresponding to an established cell.

As used herein, "transformation (cell)" refers to a phenomenon (cell) in which a spontaneous conversion into an uncontrollable growth state has occurred. Transformed cells acquire the ability to grow through infinite number of divisions in a culture vessel. Transformed cells are related to deficiency of growth control thereof and can be called with terms such as tumoral, undifferentiated and/or hyperplastic.

As used herein, "isolated" refers to at least a reduction, preferably substantially non-inclusion of a substance that is naturally present in a normal environment. Therefore, an isolated cell, tissue, or the like refers to a cell that substantially does not comprise other substances (e.g., other cells, protein, nucleic acid and the like) that accompany the cell in a natural environment.

As used herein, when referring to a certain cell, "hypertrophy of an extracellular matrix layer" an extracellular matrix layer that is thicker than an extracellular matrix layer in a normal form of the cell. For example, when referring to a human corneal endothelial cell, since the normal thickness is about 3 µm, a hypertrophy of an extracellular matrix layer is a state in which the thickness is thicker than about 3 µm, preferably the thickness is about 5 µm or more, more preferably about 6 µm or more.

As used herein, "extracellular matrix (ECM)" refers to a substance that is present between somatic cells regardless of being epithelial cells or non-epithelial cells. Extracellular matrix is produced by normal cells and is one of the substances of a living organism thereby. Extracellular matrix is not only involved in the support of tissues, but is also involved in a configuration of an internal environment that is necessary for survival of all body cells. Extracellular matrix is generally produced from a connective tissue cell. However, some are also secreted from a cell itself that holds a basal membrane such as epithelial cells or endothelial cells. Extracellular matrix components are broadly classified into fiber components and substrates filling therebetween, wherein the fiber components include collagen fiber and elastic fiber. The basic constituent component of the substrate is glycosaminoglycan (acidic mucopolysaccharide), wherein the majority of which binds with a noncollagen protein to form a polymer of proteoglycan (acidic mucopolysaccharide-protein complex). In addition, glycoprotein such as laminin of the basal membrane, microfibril of the surroundings of the elastic fiber, and fibronectin of the cell surface are also comprised in the substrate. The basic structure is also the same for specially differentiated tissue. For example, a cartilage substrate comprising a characteristically large amount of fibronectin is produced by a chondroblast in a hyaline cartilage and bone substrate wherein calcareous deposition occurs is produced by an osteoblast in bone. In this regard, a typical substance constituting extracellular matrix can include, for example, but is not limited to, collagen I, collagen IV, collagen VIII, collagen III, collagen V, elastin, vitronectin, fibronectin, laminin, thrombospondin, proteoglycans (e.g., decorin, biglycan, fibromodulin, lumican, hyaluronic acid, aggrecan and the like) and the like. As long as the extracellular matrix is responsible for cell adhesion, various kinds may be associated in the present invention.

As used herein, "extracellular matrix protein" refers to a protein constituting extracellular matrix. Such a protein can include, but is not limited to, collagen I, collagen IV, collagen VIII, collagen III, collagen V, elastin, vitronectin, fibronectin, laminin, and thrombospondin. In the present invention, at least one, preferably two, more preferably three, even more preferably four of collagen I, collagen IV, collagen VIII and fibronectin in particular can be used as indicators to determine whether or not the nature of Fuchs' endothelial corneal dystrophy is maintained.

As used herein, when referring to a certain cell, "overproduction of extracellular matrix proteins" refers to production of extracellular matrix proteins such as collagen I, collagen IV, collagen VIII and fibronectin at an amount greater than the amount produced in extracellular matrix in a normal form. The production status includes no stimulation, as well as optionally increasing the amount of expression due to a response to transforming growth factor (TGF)-β. For example, this can be about 1.1 fold or greater, about 1.2 fold or greater, about 1.3 fold or greater, about 1.4 fold or greater, about 1.5 fold or greater, about 1.6 fold or greater, about 1.7 fold or greater, about 1.8 fold or greater, about 1.9 fold or greater, or about 2.0 fold or greater with respect to the amount of extracellular matrix under normal circumstances for human corneal endothelial cells. The difference relative to normal is preferably, but not necessarily, significant.

As used herein, "epithelial mesenchymal translation (EMT)-related marker" refers to a marker that becomes an indicator in epithelial mesenchymal translation (EMT), which can include ZEB1 and Snail1, and the like.

As used herein, when referring to a certain cell, "at least one epithelial mesenchymal translation (EMT)-related marker expression rise selected from the group consisting of ZEB1 and Snail1" refers to production at an amount greater than the amount produced in ZEB1 or Snail1 in a normal form. The production status includes no stimulation, as well as optionally increasing the amount of expression due to a response to transforming growth factor (TGF)-β. For example, this can be about 1.1 fold or greater, about 1.2 fold or greater, about 1.3 fold or greater, about 1.4 fold or greater, about 1.5 fold or greater, about 1.6 fold or greater, about 1.7 fold or greater, about 1.8 fold or greater, about 1.9 fold or greater, or about 2.0 fold or greater with respect to the production amount of ZEB1 or Snail1 under normal circumstances for human corneal endothelial cells. The difference relative to normal is preferably, but not necessarily, significant.

As used herein, "non-fibroblastic form" refers to a form of a cell, wherein there is no fibroblast. Specifically, said form refers to, but is not limited to, a type of form that maintains a polygonal form as in normal cells, does not cause significant multilayering, and has a pumping function and a barrier function. Although a fibroblast does not have an outstanding function in a normal tissue, a fibroblast is a cell unique to connective tissue that upon addition of injury, migrates to the injured part and initiates production of extracellular matrix such as collagen, which renews the extracellular matrix.

As used herein, when referring to a specific cell such as a corneal endothelial cell, "normal cell function" of a cell refers to a function that the cell originally has. When referring to a corneal endothelial cell, such a function includes, but is not limited to, ZO-1 and Na⁺/K⁺ -ATPase, adaptability to corneal transplant (Matsubara M, Tanishima T: Wound-healing of the corneal endothelium in the monkey: a morphometric study, Jpn J Ophthalmol 1982, 26:264-273;Matsubara M, Tanishima T: Wound-healing of corneal endothelium in monkey:an autoradiographic study, Jpn J Ophthalmol 1983, 27:444-450;Van Horn DL, Hyndiuk RA: Endothelial wound repair in primate cornea, Exp Eye Res 1975, 21:113-124 and VanHorn DL, Sendele DD, Seideman S, Buco PJ: Regenerative capacity of the corneal endothelium in rabbit and cat, Invest Ophthalmol Vis Sci 1977, 16:597-613) and the like.

ZO-1 and Na⁺/K⁺-ATPase can be evaluated based on expression of genes by an immunological means or observing the expression at the nucleic acid level such as RT-PCR. It can be confirmed whether or not cells of interest have a normal function by confirming that Na⁺/K⁺-ATPase and ZO-1 is being expressed and/or functioning in the same degree as normal cells.

Adaptability to corneal transplant generally can also be evaluated by mechanically curetting the corneal endothelium as a bullous keratopathy model to carry out a transplant test of cultured cells in an experimental animal such as a rabbit. However, since corneal endothelial cells of a rabbit grow in vivo, the possibility of natural healing by growing corneal endothelial cells of a host cannot be denied (Matsubara M, et al., Jpn J Ophthalmol 1982, 26: 264-273; Matsubara M, et al., Jpn J Ophthalmol 1983, 27: 444-450; Van Horn DL, et al., Exp Eye Res 1975, 21: 113-124 and Van Horn DL, et al., Invest Ophthalmol Vis Sci 1977, 16: 597-613). Therefore, evaluation of survival in primates is preferable in order to evaluate a more accurate transplantation adaptability. When evaluating transplantation adaptability to humans, evaluation of adaptability is carried out after passing of, for example, at least one month, preferably at least two months, more preferably at least three months, even more preferably at least six months, even further more preferably at least twelve months in cynomolgus monkeys or the like, which are primates. Confirmation of transplantation adaptability in primates such as monkeys is especially important in application to humans.

As used herein, a "subject" refers to a target that may be targeted for the diagnosis or detection or the like of the present application (e.g., organism such as a human or organ (eye) or cells extracted from the organism, or the like).

As used herein, a "sample" refers to any substance obtained from a subject or the like, which includes, for example, ocular cell or the like. Those skilled in the art can appropriately select a preferable sample based on the description herein.

As used herein, "SV40" is an abbreviation for simian (vacuolating) virus 40 which is a type of polyoma virus which is a type of tumor virus found in primates including humans, which is utilized in research of higher animal cell vectors or carcinogenic mechanism. The detailed information can be found in http://www.nlm.nih.gov/cgi/mesh/2013/MB_cgi?field=uid&term= D027601, Taxonomy ID: 10633. The present invention uses genes (nucleic acid sequence) of the large T antigen (SV40 large T antigen) thereof in transformation.

As used herein, a "marker (substance, protein, or gene (nucleic acid))" refers to a substance that is an index for tracking a certain state (e.g., normal cell state, transformation state, disease state, disorder state, or growing ability, level of differentiation, presence, or the like) or the presence of a risk thereof. Such a marker can include gene (nucleic acid = DNA level), gene product (mRNA, protein, or the like), metabolite, enzyme and the like. The present invention can realize detection, diagnosis, preliminary detection, estimation, or pre-diagnosis regarding a certain state (e.g., disease such as differentiation disorder) using an agent, drug, factor or means specific to a marker associated with the state, or a composition, kit or system comprising the above, or the like. As used herein, a "gene product" refers to a protein or mRNA encoded by a gene. Herein, it was discovered that a gene product that does not exhibit association with an ocular cell, especially corneal endothelial cell, (i.e., a molecule such as CD98, or the like) can be used as an indicator for whether or not an ocular cell, especially corneal endothelial cell, is normal (whether or not the cell has been transformed).

As used herein, "Telomerase Reverse Transcriptase (TERT)" (hTERT for humans) is a type of telomerase reverse transcriptase, which refers to a catalytic subunit of telomerase enzyme that constitutes a significant component of a telomerase complex together with a telomerase RNA component (TERC). TERT is also called CMM9; DKCA2; DKCB4; EST2; PFBMFT1; TCS1; TP2; TRT; hEST2; hTRT. The ID thereof is Entrez Gene ID: 7015, which is NM_001193376 (human mRNA), NP_001180305 (human protein). hTERT is understood to refer not only to a protein (or a nucleic acid encoding the protein) having the amino acid sequence described in a specific accession number (and the sequence number as specifically described herein), but also to a functionally active derivative thereof, or a functionally active fragment thereof, or a homolog thereof, or a mutant encoded in a nucleic acid that hybridizes to a nucleic acid that encodes this protein under high stringency conditions or low stringency conditions.

As used herein, "Snail1" is a protein of a C2H2 type zinc finger family that controls transcription, which is one of the epithelial mesenchymal translation (EMT)-related markers. Snail1 is also called SLUGH2; SNA; SNAH; SNAIL; SNAIL1; dJ710H13.1. The ID thereof is Entrez Gene ID: 6615, which is NM_005985 (human mRNA), NP_005976 (human protein). Snail1 is understood to refer not only to a protein (or a nucleic acid encoding the protein) having the amino acid sequence described in a specific accession number (and the sequence number as specifically described herein), but also to a functionally active derivative thereof, or a functionally active fragment thereof, or a homolog thereof, or a mutant encoded in a nucleic acid that hybridizes to a nucleic acid that encodes this protein under high stringency conditions or low stringency conditions.

As used herein, "ZEB1" refers to Zinc finger E-box-binding homeobox 1, which is one of the epithelial mesenchymal translation (EMT)-related markers. ZEB1 is also called AREB6; BZP; DELTAEF1; FECD6; NIL2A; PPCD3; TCF8; ZFHEP; ZFHX1A. The ID thereof is Entrez Gene ID: 6035, which is NM_001128128 (human mRNA), NP_001121600 (human protein). ZEB1 is understood to refer not only to a protein (or a nucleic acid encoding the protein) having the amino acid sequence described in a specific accession number (and the sequence number as specifically described herein), but also to a functionally active derivative thereof, or a functionally active fragment thereof, or a homolog thereof, or a mutant encoded in a nucleic acid that hybridizes to a nucleic acid that encodes this protein under high stringency conditions or low stringency conditions.

The "derivative", "analogue", or "mutant" used herein preferably includes, without intending to be limiting, molecules comprising a substantially homologous region in a constituent protein. In various embodiments, such a molecule is at least 30%, 40%, 50%, 60%, 70%, 80%, 90%, 95%, or 99% identical compared to an amino acid sequence of the same size or a sequence aligned by a computer homology program known in the art, or a nucleic acid encoding such a molecule can hybridize with a sequence encoding a constituent protein under stringent conditions, moderately stringent conditions, or non-stringent conditions. This is a result of modifying a naturally-occurring protein by an amino acid substitution, deletion, and addition, and refers to a protein whose derivative still exhibits, although not necessarily to the same degree, the biological function of the naturally-occurring protein. For example, the biological function of such a protein can be found by a suitable and available in vitro assay that is described herein or known in the art. Although the present invention mainly discusses humans, it is understood that the discussion applies to other species such as other primate species or species of animals of other genera and it is understood that these mammalian animals are within the scope of the present invention.

As used herein, "functionally active" refers to a polypeptide, i.e., fragment or derivative, that has the structural function, control function, or biological function of a protein such as biological activity such as telomerase activity in accordance with a form to which the polypeptide, i.e., fragment or derivative, of the present invention is associated.

As used herein, "polynucleotide which hybridizes under a stringent condition" refers to commonly used, well-known conditions in the art. Such a polynucleotide can be obtained by using a method such as colony hybridization, plaque hybridization, or southern blot hybridization while using a polynucleotide selected from the polynucleotides of the present invention as a probe. Specifically, the polynucleotide refers to a polynucleotide that can be identified by using a filter with immobilized DNA from a colony or plaque and performing hybridization at 65°C in the presence of 0.7 to 1.0 M NaCl and then using an SSC (saline-sodium citrate) solution with 0.1 to 2 times concentration (composition of an SSC solution with 1 time concentration is 150 mM sodium chloride and 15 mM sodium citrate) to wash the filter under the condition of 65°C. Hybridization can be performed in accordance with the method described in experimental publications such as Molecular Cloning 2nd ed., Current Protocols in Molecular Biology, Supplement 1 to 38, DNA Cloning 1: Core Techniques, A Pracltical Approach, Second Edition, Oxford University Press (1995). In this regard, a sequence comprising only an A sequence or only a T sequence is preferably excluded from a sequence that hybridizes under stringent conditions. Thus, the polypeptides (e.g., transthyretin or the like) used in the present invention also encompass polypeptides encoded by a nucleic acid molecule that hybridizes under stringent conditions to a nucleic acid molecule encoding a polypeptide specifically described in the present invention. These low stringency conditions include hybridization for 18 to 20 hours at 40°C in a buffer comprising 35% formamide, 5xSSC, 50mM Tris-HCl(pH7.5), 5mM EDTA, 0.02% polyvinyl pyrrolidone (PVP), 0.02% BSA, 100µg/ml denatured salmon sperm DNA, and 10% (weight/volume) dextran sulfate, washing for 1 to 5 hours at 55°C in a buffer consisting of 2xSSC, 25mM Tris-HCl(pH7.4), 5mM EDTA, and 0.1% SDS, and washing for 1.5 hours at 60°C in a buffer consisting of 2xSSC, 25mM Tris-HCl(pH7.4), 5mM EDTA, and 0.1% SDS.

As used herein, a "corresponding" gene (e.g., polynucleotide sequence or molecule) refers to a gene (e.g., polynucleotide sequence or molecule) of a certain species which has or is expected to have similar action as a predetermined gene in a benchmark species for comparison. When there is a plurality of genes having such action, the corresponding gene refers to a gene having the same evolutionary origin. Hence, a gene corresponding to a certain gene may be an ortholog of such a gene. Thus, a molecule such as TERT corresponding to each molecule such as TERT of a mouse or rat can be found in a human. Such a corresponding gene can be identified by using a technique that is well known in the art. Therefore, for example, a corresponding gene in a certain animal (e.g., mouse, rat) can be found by searching a sequence database of the animal from using the sequence found in a gene accession number as a query sequence, as a benchmark gene of the corresponding gene (e.g., gene of a human).

As used herein, "expression" of a gene, a polynucleotide, a polypeptide, or the like refers to the gene or the like being subjected to a certain action in vivo to be converted into another form. Preferably, expression refers a gene, a polynucleotide, or the like being transcribed and translated into a form of a polypeptide. However, transcription to make an mRNA may also be one embodiment of expression. More preferably, such a polypeptide form can be a form (the derivative used herein) which has undergone post-translation processing.

As used herein, "transforming growth factor-β (also denoted with the abbreviation TGF-β)" is used in the same meaning as those used in the art. It is a homodimer multifunctional cytokine with a molecular weight of 25 kD exhibiting a variety of biological activity, such as being responsible for pathogenesis of various sclerotic diseases, rheumatoid arthritis, and proliferative vitreoretinopathy, being deeply involved in hair loss, suppressing the functioning of immunocompetent cells while suppressing overproduction of protease to prevent degradation of pulmonary tissue resulting in pulmonary emphysema, and suppressing cancer cell growth. "TGF-β signal" refers to a signal mediated by TGF-β, which is elicited by TGF-β. Examples of TGF-β signals include signals mediated by TGF-β2 in addition to signals mediated by TGF-β1, TGF-β3 or the like. In humans, TGF-β has three isoforms, TGF-β1 to β3, which have homology of about 70% and similar action. TGF-β is produced as an inactive latent form with a molecular weight of about 300 kD which is unable to bind to a receptor. The action thereof is exerted by being activated on a target cell surface or the surroundings thereof to become an active form that can bind to a receptor.

As used herein, "inhibiting activity against TGF-β" or "inhibiting activity against endoplasmic reticulum associated stress inducing substance" refers to activity of inhibiting apoptosis of cells due to TGF-β/endoplasmic reticulum associated stress inducing substance. "Having inhibiting activity against TGF-β" or "having inhibiting activity against endoplasmic reticulum associated stress inducing substance" refers to having caspase activity which is substantially decreased compared to a group in which cells (iFECD or iHCEC or the like) have not been contacted with a candidate compound (candidate compound non-contacted group). Furthermore, "caspase activity which is substantially decreased" refers to caspase activity which is significantly decreased in terms of statistics compared to a candidate compound non-contacted group.

As used herein, "corneal endothelial condition, disorder, or disease due to transforming growth factor (TGF)-β signals" refers to any corneal endothelial condition, disorder, or disease induced by TGF-β in corneal endothelial cells. Regarding the corneal endothelial condition, disorder, or disease due to TGF-β signals, for example, corneal endothelial conditions, disorders, or diseases due to TGF-β include, but are not limited to, Fuchs' endothelial corneal dystrophy, post-corneal transplant disorder, corneal endotheliitis, trauma, post-ophthalmic surgery disorder, post-ophthalmic laser surgery disorder, aging, posterior polymorphous dystrophy (PPD), congenital hereditary endothelial dystrophy (CHED), idiopathic corneal endothelial disorder, and cytomegalovirus corneal endotheliitis.

As used herein, "cell viability (%)" refers to the ratio of the cell count when iFCED or iHCEC are cultured in the presence of a candidate compound to the cell count when iFCED or iHCEC are cultured in the absence of said candidate compound.

As used herein, "caspase activity" refers to the ratio of caspase activity when iFCED or iHCEC are contacted with a compound used in the present invention in the presence of TGF-β (e.g., TGF-β2) or endoplasmic reticulum associated stress inducing substance to caspase activity in a TGF-β (e.g., TGF-β2) supplemented group (in the absence of candidate compound) or endoplasmic reticulum associated stress inducing substance supplemented group (in the absence of candidate compound).

As used herein, "less toxic" refers to that the cell viability is not substantially decreased in the absence of TGF-β and endoplasmic reticulum associated stress inducing substance. "Cell viability that is not substantially decreased" refers to that the cell viability has not significantly decreased compared to a group in which cells (iFCED or iHCEC or the like) have not been contacted with a candidate compound (candidate compound non-contacted group). For example, a numerical value used as a criterion can include decrease by less than 10%, decrease by less than 5%, and the like.

As used herein, "candidate compound" refers to any compound targeted for screening. Candidate compounds include, but are not limited to, a small molecule compound, peptide, protein, nucleic acid, cell, and the like, which encompasses every substance that can achieve prevention or treatment of a corneal endothelial condition, disorder, or disease.

As used herein, "endoplasmic reticulum (ER) associated stress inducing substance" refers to a substance that can induce a denatured protein. Endoplasmic reticulum stress occurs due to accumulation of denatured proteins. Excessive accumulation of denatured proteins induces apoptosis. Endoplasmic reticulum (ER) associated stress inducing substances include MG-132, thapsigargin, and tunicamycin.

As used herein, a "corneal endothelial condition, disorder, or disease due to endoplasmic reticulum (ER) associated stress" refers to any condition, disorder, or disease associated with endoplasmic reticulum (ER) stress. Examples thereof can include, but are not limited to, conditions, disorders, or diseases associated with endoplasmic reticulum (ER) stress among damage to corneal endothelial cells in Fuchs' endothelial corneal dystrophy, corneal endothelial disorder, decreased corneal endothelial density, guttae formation, hypertrophy of the Descemet's membrane, hypertrophy of a cornea, turbidity, corneal epithelial disorder, turbidity in corneal stroma, photophobia, blurred vision, visual impairment, ophthalmalgia, epiphora, hyperemia, pain, bullous keratopathy, eye discomfort, diminished contrast, glare, edema of the corneal stroma, corneal epithelial erosion, angiogenesis and the like.

A functional equivalent of the molecule such as TERT used in the present invention can be found by searching a database or the like. As used herein, "search" refers to utilizing a certain nucleic acid base sequence electronically, biologically, or by another method to find another nucleic acid base sequence having a specific function and/or property. Examples of electronic search include, but are not limited to, BLAST (Altschul et al., J. Mol. Biol. 215: 403-410 (1990)), FASTA (Pearson & Lipman, Proc. Natl. Acad. Sci., USA 85: 2444-2448 (1988)), Smith and Waterman method (Smith and Waterman, J. Mol. Biol. 147: 195-197 (1981)), Needleman and Wunsch method (Needleman and Wunsch, J. Mol. Biol. 48: 443-453 (1970)) and the like. Examples of biological search include, but are not limited to, stringent hybridization, a macroarray with a genomic DNA applied to a nylon membrane or the like or a microarray with a genomic DNA applied to a glass plate (microarray assay), PCR, in situ hybridization and the like. Herein, a gene used in the present invention is intended to include corresponding genes identified by such electronic search or biological search.

### (Description of preferred embodiments)

The preferred embodiments of the present invention are described hereinafter. It is understood that the embodiments are exemplifications of the present invention, so that the scope of the present invention is not limited to such preferred embodiments. It should be understood that those skilled in the art can refer to the following preferred embodiments to readily make modifications or changes within the scope of the present invention.

### (Immortalized Fuchs' endothelial corneal dystrophy cells)

The screening method of the present invention uses immortalized Fuchs' endothelial corneal dystrophy cells. These immortalized Fuchs' endothelial corneal dystrophy cells is outlined in Patent Literatures 1, 2 and the like, which are incorporated herein by reference as needed.

The immortalized Fuchs' endothelial corneal dystrophy cells used in the present invention comprise at least one feature selected from the group consisting of (1) hypertrophy of an extracellular matrix layer, (2) overproduction of at least one extracellular matrix protein selected from the group consisting of collagen I, collagen IV, collagen VIII and fibronectin, (3) at least one epithelial mesenchymal translation (EMT)-related marker expression rise selected from the group consisting of ZEB1 and Snail1, (4) non-fibroblastic form, and (5) normal cell function (e.g., Na⁺/K⁺-ATPase and ZO-1 are expressed and/or functioning in the same degree as normal cells). Preferably, the cells may have at least two, at least three, at least four, or all of these features. More preferably, the cells of the present invention have all of these features. In one embodiment, the extracellular matrix protein and/or EMT-related marker expression rise includes responsiveness against transforming growth factor (TGF)-β. In another embodiment, the normal cell function comprises expression of Na⁺/K⁺-ATPase and ZO-1. This is because the cells of the present invention comprises features which were unachievable in the past as model cells of immortalized Fuchs' endothelial corneal dystrophy cells and provides suitable cells for screening an agent for treating or preventing Fuchs' endothelial corneal dystrophy.

Each of the features can undergo the following evaluations.

### (1) Hypertrophy of an extracellular matrix layer

It is confirmed that the thickness is thicker than the normal cells (normally 2 to 4 µm). It can be determined whether or not cells of interest are immortalized Fuchs' endothelial corneal dystrophy cells by observing a thickness that is preferably at least 1.5-fold, more preferably at least 2-fold of normal cells.

### (2) Overproduction of at least one extracellular matrix protein selected from the group consisting of collagen I, collagen IV, collagen VIII and fibronectin

The amount is confirmed to be greater than the amount expressed in normal cells. It can be determined whether or not cells of interest are immortalized Fuchs' endothelial corneal dystrophy cells by observing the amount of expression that is preferably at least 1.5-fold, more preferably at least 2-fold of normal cells. Alternatively, whether or not the expression of extracellular matrix proteins is advanced in response to TGF-β can also be the basis for determination.

### (3) At least one epithelial mesenchymal translation (EMT)-related marker expression advancement selected from the group consisting of ZEB1 and Snail1

The amount is confirmed to be greater than the amount expressed in normal cells. It can be determined whether or not cells of interest are immortalized Fuchs' endothelial corneal dystrophy cells by observing the amount of expression that is preferably at least 1.5-fold, more preferably at least 2-fold of normal cells. Alternatively, whether or not the expression of EMT-related marker is rise in response to TGF-β can also be the basis for determination.

### (4) Non-fibroblastic form

The shape of the cells is first determined. The determination can be made by confirming that a polygonal form is maintained as in normal cells and an epithelial cell-like form of one layer is exhibited without causing significant multilayering.

### (5) Normal cell function

Expression can be confirmed by Na⁺/K⁺-ATPase and ZO-1 being immunostained or the like as in normal cells and determination can be made by the same amount of expression as normal cells.

In another aspect, the present invention provides a method for screening an agent for treating or preventing Fuchs' endothelial corneal dystrophy using the immortalized Fuchs' endothelial corneal dystrophy cells of the present invention.

### (Preparation method of the immortalized cells of Fuchs' endothelial corneal dystrophy)

The immortalized Fuchs' endothelial corneal dystrophy cells used in the present invention are produced by introducing an SV40 large T antigen and hTERT to corneal endothelial cells from a Fuchs' endothelial corneal dystrophy patient. This preparation method of immortalized Fuchs' endothelial corneal dystrophy cells are outlined in Patent Literatures 1, 2 and the like, which are incorporated herein by reference as needed.

Conventionally, an immortalized cell can escape cell aging and acquire the ability to infinitely grow while repeating the passage of a primary culture, or can impart immortalization ability by introducing a specific gene to cells of interest. However, many of such immortalized cells lose a part or all of the form or function that the cell originally had in a living organism. Thus, in a test using such an immortalized cell line, it was understood to be difficult to accurately reflect the original characteristic in a tissue from such a cell line and it was difficult to provide immortalized cells that maintain a desired feature. However, the present invention was able to provide cells with a desired feature, i.e., maintain a feature of Fuchs' endothelial corneal dystrophy and retain a function similar to normal cells (i.e., not transformed) by using the specific gene introduction technique above.

Immortalized cells that escaped cellular senescence and acquired the ability to infinitely grow while repeating the passage of a primary culture has a stably uniform characteristic, whereas many of such immortalized cells often lose a part or all of the form or function that the cells originally had in a living organism. Thus, in a test using such an immortalized cell line, it was difficult to accurately reflect the original characteristic in a tissue from such a cell line. Culture (Zaniolo K, et al. Exp Eye Res.; 94(1):22-31. 2012) and immortalization (Azizi B, et al. Invest Ophthalmol Vis Sci. 2; 52(13): 9291-7.2011) of corneal endothelial cells from Fuchs' corneal dystrophy patients have been reported, but cells suitable for screening of a therapeutic drug or progression preventing drug which maintain the features of the disease, such as overproduction of extracellular matrices, have not been reported. Meanwhile, the present invention maintains a characteristic of overproduction of extracellular matrices, which is a feature of corneal endothelial cells in Fuchs' corneal dystrophy patients. Past attempts were to transform cells by introducing carcinogenic genes such as ras genes or c-myc genes, E1A genes of an adenovirus, large T antigen genes of an SV40 virus, HPV16 genes of human papillomavirus, or the like to primary cells and continuously retain the active growth ability of the primary cells for further passaging to establish immortalized cells that do not lose the characteristic unique to the cells. However, even in such immortalized cells, it is difficult to obtain immortalized cells in the strict sense of retaining the original function since, depending on the organ or interest, some functions are already lost at the point when primary cells are prepared and these carcinogenic genes or large T antigen genes are introduced. In particular, it is extremely difficult to prepare and establish a cell line of a cell of a special disease or a primary cell that is very slow in growth speed or from a micro organ. Furthermore, there was also a similar difficulty for Fuchs' endothelial corneal dystrophy. In particular, regarding Fuchs' endothelial corneal dystrophy, while it was difficult to maintain normal cells in corneal endothelium to begin with, it was even more difficult to create a pathology model and maintain the model as cultured cells. Thus, it was considered impossible to produce a pathology model that keeps a normal function.

In this regard, immortalization while maintaining the feature of Fuchs' endothelial corneal dystrophy was achieved by introducing Telomerase Reverse Transcriptase (TERT) as an introduction gene in addition to using an SV40 large T antigen. Such immortalized Fuchs' endothelial corneal dystrophy cells can bear screening of useful substances such as pharmaceutical products targeting Fuchs' endothelial corneal dystrophy.

Any technique can be utilized as a method for introducing an SV40 large T antigen and hTERT as long as the technique is a gene (nucleic acid molecule) introduction technique (transformation, transduction, transfection, or the like) or a gene product (protein) introduction method that is known in the art. Introduction of these genes can normally be realized by introducing a nucleic acid molecule. As used herein, "gene introduction" refers to introduction of an exogenous gene, preferably a functional gene, to, for example, a chromosomal genome or the like with an appropriate introduction technique. In addition, an exogenous functional gene can be introduced by substitution with an endogenous functional gene by utilizing a targeted genetic modification method that is known in the art. Furthermore, the exogenous functional gene is a gene that is originally absent from the chromosomal genome of the living organism, which may be genes such as genes from other living species, or synthetic genes produced with PCR or the like. For example, a transfection method may be included as a method for introducing such a gene. Such a transfection introduces a gene of interest (an SV40 large antigen, an hTERT gene and the like in the present invention) is introduced to an appropriate viral vector (e.g., but is no limited to, a lentiviral vector). A helper plasmid (e.g., pLP1, pLP2, pLP/VSVG, or the like) was then used as needed to infect suitable cells (e.g., 293T cell). After culturing under an appropriate condition, culture supernatant comprising viruses was collected after the infection. This culture supernatant was added to a culture solution of the cells of interest (cultured corneal endothelial cells from a Fuchs' endothelial corneal dystrophy patient in the present invention) together with an appropriate reagent (which was, but is not limited to, polybrene) to enable production of an immortalized corneal endothelial cell line (iFECD) from a Fuchs' endothelial corneal dystrophy patient. Alternatively, as another example, an exogenous DNA comprising a DNA that encodes transformation genes may be introduced to cells of interest using a technique of retrovirus transfection (e.g., lentiviral vector). For example, a recombinant retrovirus is produced in a packaging cell line and produces a culture supernatant that has a high titer of viral particles (generally, but is not limited to, 10⁵ to 10⁶ pfu/ml). Recombinant viral particles are used to infect a culture of cells of interest by incubating the cell culture with a medium comprising viral particles and a reagent such as polybrene at an appropriate concentration (e.g., but is not limited to, 5 to 10 µg/ml) for an appropriate amount of time (e.g., but is not limited to, about 1 to 12 hours). After being infected with the retrovirus, the cells are rinsed and cultured in a standard medium. Next, infected cells will be analyzed for intake and expression of the exogenous DNA. The cells can be subjected to a selective condition for selecting cells that took in and expressed a selective marker gene. Alternatively, exogenous DNA can be introduced using a technique of calcium phosphate-mediation-type transfection. A gene of interest, e.g., a calcium phosphate precipitate comprising a DNA that encodes transformation genes, may be prepared using the technique of Wigler et al. (1979) Proc. Natl. Acad. Sci. USA 76: 1373-1376. After the transfection, the cells are analyzed for intake and expression of the exogenous DNA. The cells can be subjected to a selective condition for selecting cells that took in and expressed a selective marker gene. Such a technique is exemplified in, but not limited to, Japanese National Phase PCT Laid-Open Publication No. 2013-509179 and Japanese Laid-Open Publication No. 2004-254509, other than in the specifically disclosed examples in the Examples.

The following conditions can be included as detailed conditions that are useful if added upon immortalization. For example, in a preferable embodiment, cells can be obtained by culturing the corneal endothelial cells from said Fuchs' endothelial corneal dystrophy patient in a medium comprising SB431542 (4-[4-(1,3-benzodioxole-5-yl)-5-(2-pyridinyl)-1H-imidazole-2-yl]benzamide) and SB203580 (4-[4-(4-fluorophenyl)-2-(4-methylsulfinylphenyl)-1H-imidazole-5-yl]pyridine). This medium may preferably be, but is not limited to, the SB203580 + SB431542 + 3T3 conditioned medium that was used in the Example.

### (Screening method using immortalized Fuchs' endothelial corneal dystrophy cells)

In one aspect, the present invention provides a method of screening a compound that is effective against a corneal endothelial disease, using immortalized Fuchs' endothelial corneal dystrophy cells (iFECD). The method of the present invention comprises primary screening that selects a compound having inhibiting activity against TGF-β and secondary screening that selects a compound with low toxicity to cells and inhibiting activity against TGF-β. The present invention carries out both of the primary screening and the secondary screening to provide a highly reliable screening method. In a specific embodiment, the method of the present invention may comprise (a) a step of contacting a candidate compound with immortalized Fuchs' endothelial corneal dystrophy cells in the presence of transforming growth factor (TGF)-β to evaluate inhibiting activity of the candidate compound against the TGF-β and selecting a compound having the inhibiting activity, (b) a step of evaluating toxicity of the compound selected in step (a) against the cells, (c) a step of evaluating inhibiting activity of the compound selected in step (a) against the TGF-β, if necessary, and (d) a step of selecting a compound evaluated to be less toxic to the cells in step (b) and evaluated to have inhibiting activity against TGF-β in step (c).

The primary screening corresponds to (a) the step of contacting a candidate compound with immortalized Fuchs' endothelial corneal dystrophy cells in the presence of transforming growth factor (TGF)-β to evaluate the inhibiting activity of the candidate compound against the TGF-β and selecting a compound having the inhibiting activity and the secondary screening corresponds to (b) the step of evaluating toxicity of the compound selected in step (a) to the cells, (c) the step of evaluating inhibiting activity of the compound selected in step (a) against the TGF-β, if necessary, and (d) the step of selecting a compound evaluated to be less toxic to the cells in step (b) and evaluated to have the inhibiting activity against the TGF-β in step (a) or (c). Although step (c) is not an essential step, it is preferable to comprise step (c) in order to improve precision of the screening. Surprisingly, this is because the precision of the screening significantly raised by carrying out step (c), which was unexpected. Since the increase is significant even after considering the mere mastery of testers and other factors, it is understood to be advantageous to comprise step (c). The effect by encompassing such step (c) could not have been expected from conventional screening techniques.

In one embodiment, the number of procedures of evaluation of the inhibiting activity against the TGF-β in step (c) may be larger than the number of procedures of evaluation of the inhibiting activity against the TGF-β in step (a). This is for selecting a more highly reliable and useful compound by increasing the number of procedures in step (c) in the secondary screening. There is also an advantage of enabling the processing of a large amount of candidate compounds by setting the number of procedures of the primary screening to be somewhat smaller. Due to the small number of procedures, there is a possibility of lowering reliability of the primary screening. However, reliability is guaranteed by the secondary screening. In fact, some of the compounds that were considered useful in the primary screening were removed in the secondary screening. Table 1 shows an example wherein although caspase activity of 75% or less was exhibited in the primary screening, a decrease of caspase activity was hardly found in the secondary screening with an increased number of procedures. Table 1 shows 90% or more cell viability in "○" and less than 90% cell viability in "×", which were measured with CellTiter-Glo® Luminescent Cell Viability Assay (Promega catalog number: G7570).

**[Table 1]**

| **Plate Location** | **Name** | Primary screening Caspase 3/7 activity (%) | Secondary screening Caspase 3/7 activity (%) | | Secondary screening Viable cell number (%) | | Caspase 3/7 activity /Viable cell number (%) | |
|---|---|---|---|---|---|---|---|---|
| 6-C11 | Asenapine Maleate | 72.85 | × | 88.85 | ○ | 101.64 | × | 87.42 |
| 3-B10 | Letrozole | 71.2 | × | 103.7 | ○ | 101.3 | × | 102.43 |
| 6-A05 | Acebutolol ·HCl | 74.30 | × | 88.05 | ○ | 99.77 | × | 88.25 |
| 5-A09 | Primaquine Phosphate | 74.31 | × | 99.08 | ○ | 99.7 | × | 99.37 |
| 5-E07 | Milrinone | 62.45 | × | 94.88 | ○ | 99.67 | × | 95.20 |
| 5-C06 | Tetracycline | 60.12 | × | 93.69 | ○ | 99.31 | × | 94.34 |

In some embodiments, the number of procedures of evaluation of the inhibiting activity against the TGF-β in step (a) may be 1 time, 2 times, 3 times, 4 times, 5 times, or more times, and the number of procedures of evaluation of the inhibiting activity against the TGF-β in step (c) may be 2 times, 3 times, 4 times, 5 times, 6 times, 7 times, 8 times, 9 times, 10 times, or more times so as to exceed the number of procedures in step (a). In a specific embodiment, the number of procedures of evaluation of the inhibiting activity against the TGF-β in step (c) may be n = 4 to 6 and the number of procedures of evaluation of the inhibiting activity against the TGF-β in step (a) may be n = 1 to 3. In a preferable embodiment, the number of procedures of evaluation of the inhibiting activity against the TGF-β in step (c) may be n = 5 and the number of procedures of evaluation of the inhibiting activity against the TGF-β in step (a) may be n = 2. Those skilled in the art can consider the number of candidate compounds and set an appropriate number of procedures.

In some embodiments, inhibiting activity against TGF-β may be evaluated based on activity of inhibiting apoptosis of cells due to a TGF-β signal. The activity of inhibiting apoptosis may be evaluated with, for example, caspase activity (e.g., Caspase 3/7 activity), exposure of phosphatidylserine in the outer surface of the cell membrane, cleavage of Poly(ADP-ribose)Polymerase(PARP), or fragmentation of chromosomal DNA as an indicator. In a specific embodiment, inhibiting activity against TGF-β may be evaluated by measuring caspase activity (e.g., Caspase 3/7 activity) and, for example, a compound with substantially decreased caspase activity compared to a candidate compound non-contact group may be selected as a compound having inhibiting activity against TGF-β. A candidate compound non-contact group is a group to which a candidate compound is not supplemented (TGF-β supplemented group). Caspase activity (e.g., Caspase 3/7 activity) which is substantially decreased compared to a candidate compound non-contact group is the caspase activity (e.g., Caspase 3/7 activity) that significantly decreased in terms of statistics compared to a candidate compound non-contact group, which may be, for example, caspase activity (e.g., Caspase 3/7 activity) that is about 95% or less, about 90% or less, about 85% or less, about 80% or less, about 75% or less, about 70% or less, about 65% or less, about 60% or less, or about 55% or less compared to a candidate compound non-contact group. In a certain embodiment, a compound exhibiting about 75% or less Caspase 3/7 activity compared to a candidate compound non-contact group may be selected as a compound having inhibiting activity against TGF-β.

In the secondary screening, toxicity to immortalized Fuchs' endothelial corneal dystrophy cells of a candidate compound may be evaluated. The evaluation of toxicity enables selection of a compound that is useful as a medicament. The toxicity may be evaluated based on cell viability.

In one embodiment, step (b) comprises measurement of cell viability. When the cell viability is not substantially decreased, the toxicity will be evaluated to be low. The cell viability is measured in the absence of TGF-β. Cell viability that is not substantially decreased means that the cell viability is not significantly decreased compared to a candidate compound non-contact group, which may be, for example, cell viability that is about 90% or more compared to a candidate compound non-contact group. The threshold value of the cell viability may be about 80%, about 85%, about 90%, about 95%, about 98%, about 99%, about 100%. It has been discovered that there is a case in which the toxicity is strong and the cell number is significantly decreased even if caspase activity appeared to be suppressed since caspase activity is decreased due to decrease in cell number. Thus, a drug can be accurately selected by measuring cell number in addition to measuring caspase activity. Although the compounds shown in Table 2 appeared to be suppressing caspase activity, the compounds had strong toxicity, wherein cell viability went below 90%, and were thereby removed in the secondary screening. Table 2 shows 90% or more cell viability in "○" and less than 90% cell viability in "×", which were measured with CellTiter-Glo® Luminescent Cell Viability Assay (Promega catalog number: G7570).

**[Table 2]**

| **Plate Location** | **Name** | Primary screening Caspase 3/7 activity (%) | Secondary screening Caspase 3/7 activity (%) | Secondary screening Viable cell number (%) | | Caspase 3/7 activity /Viable cell number (%) | |
|---|---|---|---|---|---|---|---|
| 9-E10 | Nitazoxanide | 36.53 | 43.31 | × | 87.81 | × | 49.32 |
| 9-H08 | Pimecrolimus | 70.14 | 52.97 | × | 84.64 | × | 62.58 |
| 7-D07 | Deferasirox | 49.96 | 38.61 | × | 56.04 | × | 68.90 |
| 3-D04 | Mycophenolate Mofetil | 46.4 | 47.6 | × | 67.7 | × | 70.36 |
| 2-B11 | Carvedilol | 50.3 | 64.1 | × | 89.1 | × | 71.9 |

The secondary screening may select a compound exhibiting cell viability that is not substantially decreased and Caspase 3/7 activity that is substantially decreased compared to a candidate compound non-contact group. Such a compound may be a compound exhibiting Caspase 3/7 activity (%) ratio of about 0.9 or less, about 0.8 or less, about 0.75 or less, about 0.7 or less, about 0.65 or less, about 0.6 or less, about 0.55 or less, about 0.5 or less, about 0.45 or less, about 0.4 or less, about 0.35 or less, about 0.3 or less, about 0.25 or less, or about 0.2 or less to the cell viability (%). In a specific embodiment, a compound exhibiting Caspase 3/7 activity (%) ratio of about 0.8 or less to the cell viability (%) may be selected in step (d).

In the screening method of the present application, the concentration of a candidate compound may be at any concentration. It is understood that the same concentration may be used for all of the concentrations of candidate compounds, or different concentrations may be used for every candidate compound. When a concentration that exhibits toxicity to cells (e.g., 50% inhibition concentration: IC50) is known, those skilled in the art would be able to set the concentration of a candidate compound with said concentration in mind. The concentration of a candidate compound to be used includes, for example, but is not limited to, about 1 nM, about 10 nM, about 100 nM, about 1 µM, about 10 µM, about 100 µM, about 1 mM, about 10 mM, and about 100 mM. Any numerical value between these concentrations (e.g., about 2, about 3, about 4, about 5, about 6, about 7, about 8, or about 9×10¹, 10², 10³, 10⁴, 10⁵, 10⁶, 10⁷, or 10⁸ nM) can also be used. A range between any points of these numerical values can also be employed as a range of preferable concentrations in the present invention. In a specific embodiment, the concentration of a candidate compound is about 10 µM. In another embodiment, the concentration of a candidate compound is about 1 µM.

Since the screening method of the present invention selects a compound having inhibiting activity against TGF-β, a compound selected by the screening method of the present invention may prevent or treat a corneal endothelial condition, disorder, or disease due to TGF-β signals. The corneal endothelial condition, disorder, or disease due to TGF-β may be selected from the group consisting of Fuchs' endothelial corneal dystrophy, post-corneal transplant disorder, corneal endotheliitis, trauma, post-ophthalmic surgery disorder, post-ophthalmic laser surgery disorder, aging, posterior polymorphous dystrophy (PPD), congenital hereditary endothelial dystrophy (CHED), idiopathic corneal endothelial disorder, and cytomegalovirus corneal endotheliitis. Since the screening method of the present invention uses immortalized Fuchs' endothelial corneal dystrophy cells, a compound selected by the screening method of the present invention may prevent or treat Fuchs' endothelial corneal dystrophy in particular.

In another embodiment, a compound selected by a screening method using immortalized Fuchs' endothelial corneal dystrophy cells can further be provided in the screening method using immortalized human corneal endothelial cells, which is discussed in detail below. Alternatively, a compound selected by the screening method using immortalized human corneal endothelial cells can be provided in the screening method using immortalized Fuchs' endothelial corneal dystrophy cells. When using these two types of screening systems, the second screening system may omit the primary screening as needed. This is because the number of candidate compounds is limited to a certain extent by the first screening. Since the screening method using immortalized human corneal endothelial cells selects a compound having inhibiting activity against an endoplasmic reticulum associated stress inducing substance, a compound selected by a screening method using both immortalized Fuchs' endothelial corneal dystrophy cells and immortalized human corneal endothelial cells may have inhibiting activity against TGF-β and endoplasmic reticulum associated stress inducing substance. Since Fuchs' endothelial corneal dystrophy is considered to be one of the causes of endoplasmic reticulum associated stress, a compound having inhibiting activity against TGF-β and endoplasmic reticulum associated stress inducing substance is considered especially effective for Fuchs' endothelial corneal dystrophy. In addition, it was revealed that a compound may not exhibit any inhibiting activity against an endoplasmic reticulum associated stress inducing substance at all even if the compound was shown to have inhibiting activity against TGF-β. This shows that it is possible to screen a compound effective against a corneal endothelial disorder or the like (e.g., Fuchs' endothelial corneal dystrophy) due to both TGF-β and endoplasmic reticulum associated stress inducing substance by using a combination of the two screenings. A typical schematic diagram of a screening method using both immortalized Fuchs' endothelial corneal dystrophy cells and immortalized human corneal endothelial cells is shown in Figure **1****.**

### (Screening method using immortalized human corneal endothelial cells)

In one aspect, the present invention provides a method of screening a compound that is effective against a corneal endothelial disease, using immortalized human corneal endothelial cells (iHCEC). The method of the present invention comprises primary screening that selects a compound having inhibiting activity against an endoplasmic reticulum (ER) associated stress inducing substance and secondary screening that selects a compound with low toxicity to cells and inhibiting activity against an endoplasmic reticulum (ER) associated stress inducing substance. The use of immortalized human corneal endothelial cells (iHCEC) induced by an endoplasmic reticulum (ER) associated stress inducing substance enables screening of a compound effective against a corneal endothelial disease from the viewpoint of endoplasmic reticulum (ER) associated stress, which is different from a screening method using iFECD. The present invention carries out both of the primary screening and the secondary screening to provide a highly reliable screening method. In a specific embodiment, the method of the present invention may comprise (a) a step of contacting a candidate compound with immortalized human corneal endothelial cells in the presence of an endoplasmic reticulum (ER) associated stress inducing substance to evaluate inhibiting activity of the candidate compound against the endoplasmic reticulum (ER) associated stress inducing substance and selecting a compound having the inhibiting activity, (b) a step of evaluating toxicity of the compound selected in step (a) against the cells, (c) a step of evaluating inhibiting activity of the compound selected in step (a) against the endoplasmic reticulum (ER) associated stress inducing substance, and (d) a step of selecting a compound evaluated to be less toxic to the cells in step (b) and evaluated to have inhibiting activity against the endoplasmic reticulum (ER) associated stress inducing substance in step (c).

An endoplasmic reticulum associated stress inducing substance includes MG-132, thapsigargin and tunicamycin. MG-132 is known as a substance that can induce abnormal folding of proteins (accumulation of unfolded proteins), which is one of the causes of endoplasmic reticulum associated stress. Especially, since endoplasmic reticulum associated stress due to accumulation of unfolded proteins is known to be one of the causes of Fuchs' endothelial corneal dystrophy, endoplasmic reticulum associated stress induced by MG-132 is considered to reflect the state of Fuchs' endothelial corneal dystrophy even more. A screening method that evaluates inhibiting activity against MG-132 using iHCEC can screen a compound that suppresses the pathology of Fuchs' endothelial corneal dystrophy (endoplasmic reticulum associated stress caused by accumulation of unfolded proteins) that cannot be evaluated with a screening method using the above iFCED, which is advantageous. Although not wishing to be bound by any theory, it can actually be seen from the screened compound that a compound, wherein inhibiting activity against MG-132 was confirmed, is actually a compound that advantageously suppresses the pathology of Fuchs' endothelial corneal dystrophy.

The primary screening corresponds to (a) the step of contacting a candidate compound with immortalized human corneal endothelial cells in the presence of an endoplasmic reticulum (ER) associated stress inducing substance to evaluate the inhibiting activity of the candidate compound against the endoplasmic reticulum (ER) associated stress inducing substance and selecting a compound having the inhibiting activity and the secondary screening corresponds to (b) the step of evaluating toxicity of the compound selected in step (a) to the cells, (c) the step of evaluating inhibiting activity of the compound selected in step (a) against the endoplasmic reticulum (ER) associated stress inducing substance, and (d) the step of selecting a compound evaluated to be less toxic to the cells in step (b) and evaluated to have the inhibiting activity against the endoplasmic reticulum (ER) associated stress inducing substance in step (c).

In one embodiment, the number of procedures of evaluation of the inhibiting activity against an endoplasmic reticulum (ER) associated stress inducing substance in step (c) may be larger than the number of procedures of evaluation of inhibiting activity against an endoplasmic reticulum (ER) associated stress inducing substance in step (a). This is for selecting a more highly reliable and useful compound by increasing the number of procedures in step (c) in the secondary screening. There is also an advantage of enabling the processing of a large amount of candidate compounds by setting the number of procedures of the primary screening to be somewhat smaller. Due to the small number of procedures, there is a possibility of lowering reliability in the primary screening. However, reliability is guaranteed by the secondary screening. In some embodiments, the number of procedures of evaluation of inhibiting activity against an endoplasmic reticulum (ER) associated stress inducing substance in step (a) may be 1 time, 2 times, 3 times, 4 times, 5 times, or more times, and the number of procedures of evaluation of inhibiting activity against an endoplasmic reticulum (ER) associated stress inducing substance in step (c) may be 2 times, 3 times, 4 times, 5 times, 6 times, 7 times, 8 times, 9 times, 10 times, or more times so as to exceed the number of procedures in step (a). In a specific embodiment, the number of procedures of evaluation of inhibiting activity against an endoplasmic reticulum (ER) associated stress inducing substance in step (c) may be n = 4 to 6 and the number of procedures of evaluation of inhibiting activity against an endoplasmic reticulum (ER) associated stress inducing substance in step (a) may be n = 1 to 3. In a preferable embodiment, the number of procedures of evaluation of inhibiting activity against an endoplasmic reticulum (ER) associated stress inducing substance in step (c) may be n = 5 and the number of procedures of evaluation of inhibiting activity against an endoplasmic reticulum (ER) associated stress inducing substance in step (a) may be n = 2. Those skilled in the art can consider the number of candidate compounds and set an appropriate number of procedures.

In some embodiments, inhibiting activity against endoplasmic reticulum (ER) associated stress inducing substance may be evaluated based on activity of inhibiting apoptosis of cells due to an endoplasmic reticulum (ER) associated stress inducing substance. The activity of inhibiting apoptosis may be evaluated with, for example, caspase activity (e.g., Caspase 3/7 activity), exposure of phosphatidylserine in the outer surface of the cell membrane, cleavage of Poly(ADP-ribose)Polymerase(PARP), or fragmentation of chromosomal DNA as an indicator. In a specific embodiment, the inhibiting activity against an endoplasmic reticulum (ER) associated stress inducing substance may be evaluated by measuring caspase activity (e.g., Caspase 3/7 activity) and, for example, a compound with substantially decreased caspase activity compared to a candidate compound non-contact group may be selected as a compound having inhibiting activity against an endoplasmic reticulum (ER) associated stress inducing substance. A candidate compound non-contact group is a group to which a candidate compound is not supplemented (endoplasmic reticulum (ER) associated stress inducing substance supplemented group). Caspase activity which is substantially decreased compared to a candidate compound non-contact group is the caspase activity (e.g., Caspase 3/7 activity) that significantly decreased in terms of statistics with respect to a candidate compound non-contact group, which may be, for example, caspase activity (e.g., Caspase 3/7 activity) that is about 95% or less, about 90% or less, about 85% or less, about 80% or less, about 75% or less, about 70% or less, about 65% or less, about 60% or less, or about 55% or less with respect to a candidate compound non-contact group. In a certain embodiment, a compound showing about 75% or less Caspase 3/7 activity compared to a candidate compound non-contact group may be selected as a compound having inhibiting activity against an endoplasmic reticulum (ER) associated stress inducing substance.

In the secondary screening, toxicity to immortalized human corneal endothelial cells of a candidate compound may be evaluated. The evaluation of toxicity enables selection of a compound that is useful as a medicament. The toxicity is may be evaluated based on cell viability.

In one embodiment, step (b) comprises measurement of cell viability. When the cell viability is not substantially decreased, the toxicity will be evaluated to be low. The cell viability is measured in the absence of an endoplasmic reticulum (ER) associated stress inducing substance. Cell viability that is not substantially decreased means that the cell viability is not significantly decreased compared to a candidate compound non-contact group, which may be, for example, cell viability that is about 90% or more compared to a candidate compound non-contact group. The threshold value of the cell viability may be about 80%, about 85%, about 90%, about 95%, about 98%, about 99%, about 100%. It has been discovered that there is a case in which the toxicity is strong and the cell number is significantly decreased even if caspase activity appears to be suppressed since caspase activity is decreased due to decrease of cell number. Thus, a drug can be accurately selected by measuring cell number in addition to measuring caspase activity.

The secondary screening may select a compound showing cell viability that is not substantially decreased and Caspase 3/7 activity which is substantially decreased compared to a candidate compound non-contact group. Such a compound may be a compound showing Caspase 3/7 activity (%) ratio of about 0.9 or less, about 0.85 or less, about 0.8 or less, about 0.75 or less, about 0.7 or less, about 0.65 or less, about 0.6 or less, about 0.55 or less, about 0.5 or less, about 0.45 or less, about 0.4 or less, about 0.35 or less, about 0.3 or less, about 0.25 or less, or about 0.2 or less compared to the cell viability (%). In a specific embodiment, a compound showing Caspase 3/7 activity (%) ratio of about 0.8 or less compared to the cell viability (%) may be selected in step (d).

Since the screening method of the present invention selects a compound having inhibiting activity against an endoplasmic reticulum (ER) associated stress inducing substance, a compound selected by the screening method of the present invention may prevent or treat a corneal endothelial condition, disorder, or disease due to an endoplasmic reticulum (ER) associated stress inducing substance. The corneal endothelial condition, disorder, or disease due to an endoplasmic reticulum (ER) associated stress inducing substance may be caused by abnormal folding of proteins. In a specific embodiment, the corneal endothelial condition, disorder, or disease due to an endoplasmic reticulum (ER) associated stress inducing substance may be the damage to corneal endothelial cells in Fuchs' endothelial corneal dystrophy, corneal endothelial disorder, decreased corneal endothelial density, guttae formation, hypertrophy of the Descemet's membrane, hypertrophy of a cornea, turbidity, corneal epithelial disorder, turbidity in corneal stroma, photophobia, blurred vision, visual impairment, ophthalmalgia, epiphora, hyperemia, pain, bullous keratopathy, eye discomfort, diminished contrast, glare, edema of the corneal stroma, and corneal epithelial erosion. Since endoplasmic reticulum (ER) associated stress is known as one of the causes of Fuchs' endothelial corneal dystrophy, a compound selected by the screening method of the present invention may prevent or treat Fuchs' endothelial corneal dystrophy in particular.

### (Kit)

In another aspect, the present invention provides a kit for screening a compound effective against a corneal endothelial disease using the screening method above.

As used herein, "kit" refers to a unit providing portions to be provided (e.g., agent, antibody, label, cell, manual, and the like), generally in two or more separate sections. This form of a kit is preferred when intending to provide a composition that should not be provided in a mixed state but is preferably mixed immediately before use for safety reasons or the like. Such a kit advantageously comprises instructions or a manual preferably describing how the provided portions (e.g., agent) should be used or how a reagent should be handled. When the kit is used herein as a reagent kit, the kit generally comprises an instruction describing how to use an agent, antibody, and the like.

As used herein, "instruction" is a document with an explanation of the method of use of the present invention for a user. The instruction describes a screening method of the invention and use method of a reagent. Further, an instruction may have a description instructing a use method (screening method). The instruction is prepared in accordance with a format specified by a regulatory authority of the country in which the invention is practiced (e.g., Ministry of Health, Labour and Welfare in Japan, Food and Drug Administration (FDA) in the U.S., or the like), with an explicit description showing approval by the regulatory authority. The instruction is a so-called package insert, and may be provided in paper media. The instructions may also be provided in a form such as electronic media (e.g., web sites provided on the Internet or emails).

In a specific embodiment, the kit of the present invention may comprise TGF-β, immortalized Fuchs' endothelial corneal dystrophy cells, reagent or device for measuring inhibiting activity against TGF-β, and reagent or device for measuring cell toxicity. TGF-β may be TGF-β2. The preparation method of immortalized Fuchs' endothelial corneal dystrophy cells is as explained above.

In another embodiment, the kit of the present invention may comprise an endoplasmic reticulum associated stress inducing substance, immortalized human corneal endothelial cells, reagent or device for measuring inhibiting activity against an endoplasmic reticulum associated stress inducing substance, and reagent or device for measuring cell toxicity. An endoplasmic reticulum associated stress inducing substance include, for example, MG-132, thapsigargin and tunicamycin. In a specific embodiment, the endoplasmic reticulum associated stress inducing substance may be MG-132. The immortalized human corneal endothelial cells were prepared using a method similar to immortalized Fuchs' endothelial corneal dystrophy cells (iFECD), using a cornea for research (SightLife) from a normal person.

In another different embodiment, the kit of the present invention may comprise TGF-β, an endoplasmic reticulum associated stress inducing substance, immortalized Fuchs' endothelial corneal dystrophy cells, immortalized human corneal endothelial cells, reagent or device for measuring inhibiting activity against TGF-β, reagent or device for measuring inhibiting activity against an endoplasmic reticulum associated stress inducing substance, and reagent or device for measuring cell toxicity.

The reagent or device of the present invention may comprise a "label" for identifying a molecule or substance of interest among others. Other than a substance (fluorescent substance, luminescent substance, or the like), a "label" also comprise energy, electromagnetic wave, radioactive ray, or the like. A labeling method can include, RI (radioisotope) method, fluorescence method, biotin method, chemiluminescence method, and the like. Upon labeling, anything can be used as long as the function is not affected.

In some embodiments, a reagent or device for measuring inhibiting activity of TGF-β or an endoplasmic reticulum associated stress inducing substance may be a reagent or device for measuring caspase activity (e.g., Caspase 3/7 activity). The reagent or device for measuring inhibiting activity of TGF-β or an endoplasmic reticulum associated stress inducing substance may comprise a luminescence reagent. The luminescence reagent may be a reagent to enable a target (which is caspase in a case of caspase activity) to be measured luminescent. The reagent or device for measuring caspase activity (e.g., Caspase 3/7 activity) may include, but is not limited to, a reagent or device used in Caspase-Glo® 3/7 Assay, CellEvent™ Caspase-3/7 Green Detection Reagent, SensoLyte® Homogeneous AFC Caspase-3/7 Assay Kit, or the like. Those skilled in the art can use a known technique, a commercially available kit, or reagent or device, for measuring caspase activity (e.g., Caspase 3/7 activity).

In some embodiments, a reagent or device for measuring cell toxicity may be a device for measuring cell viability. The reagent or device for measuring cell toxicity may be a luminescence reagent. The luminescence reagent may be a reagent to enable a target (which is living cells, dead cells, or both in a case of cell viability) to be measured luminescent. The reagent or device for measuring cell viability may include, but is not limited to, a reagent or device used in CellTiter-Glo® Luminescent Cell Viability Assay, RealTime-Glo™ MT Cell Viability Assay, CellTiter-Blue® Cell Viability Assay, AlamarBlue™ Cell Viability Reagent, or the like. Those skilled in the art can use a known technique, a commercially available kit, or reagent or device, for measuring cell viability.

Reference literature such as scientific literature, patents, and patent applications cited herein is incorporated herein by reference to the same extent that the entirety of each document is specifically described.

The present invention has been explained while showing preferred embodiments to facilitate understanding. The present invention is explained hereinafter based on Examples. The above explanation and the following Examples are not provided to limit the present invention, but for the sole purpose of exemplification. Thus, the scope of the present invention is not limited to the embodiments and Examples that are specifically disclosed herein and is limited only by the scope of claims.

### [Examples]

Hereinafter, examples of the present invention are described. Biological samples or the like, where applicable, were handled in compliance with the standards enacted by the Ministry of Health, Labour and Welfare, Ministry of Education, Culture, Sports, Science and Technology, or the like and, where applicable, based on the Helsinki Declaration or ethical codes prepared based thereon. For the donation of eyes used for the study, consent was obtained from close relatives of all deceased donors. The present study was approved by the ethics committee or a corresponding body of the University of Erlangen-Nuremberg (Germany) and SightLife™ (Seattle, WA) eye bank.

### (Example 1: Production of Fuchs' endothelial corneal dystrophy patient derived immortalized corneal endothelial cell line (iFECD) and immortalized corneal endothelial cells of normal corneal endothelial cells (iHCEC))

In this example, immortalized corneal endothelial cell lines (iFECD and iHCEC) were made from corneal endothelial cells from Fuchs' endothelial corneal dystrophy patients and healthy subjects, respectively.

### (Culture method)

Corneal endothelial cells were mechanically peeled off together with a basal membrane from a cornea for research purchased from the Seattle Eye Bank. After using collagenase to detach and collect the corneal endothelial cells from the basal membrane, the cells were subjected to primary culture. As for a medium, Opti-MEM I Reduced-Serum Medium, Liquid (INVITROGEN catalog number: 31985-070), to which 8% FBS (BIOWEST, catalog number: S1820-500), 200 mg/ml of CaCl₂·2H₂O (SIGMA catalog number: C7902-500G), 0.08% of chondroitin sulfate (SIGMA catalog number: C9819-5G), 20 µg/ml of ascorbic acid (SIGMA catalog number: A4544-25G), 50 µg/ml of gentamicin (INVITROGEN catalog number: 15710-064) and 5 ng/ml of EGF (INVITROGEN catalog number: PHG0311) were added, and conditioned for 3T3 feeder cells were used as a basal medium. Further, the cells were cultured in a basal medium to which SB431542 (1 µmol/l) and SB203580 (4-(4-fluorophenyl)-2-(4-methylsulfonylphenyl)-5(4-pyridyl) imidazole<4-[4-(4-fluorphenyl)-2-(4-methylsulfinylphenyl)-1 H-imidazole-5-yl]pyridine) (1 µmol/l) were added (referred to as "SB203580 + SB431542 + 3T3 conditioned medium").

### (Method of acquisition)

Corneal endothelial cells were obtained with approval from an ethics committee and written consent from 3 human patients who suffered from bullous keratopathy according to a clinical diagnosis of Fuchs' endothelial corneal dystrophy and underwent corneal endothelial transplant (Descemet's Membrane Endothelial Keratoplasty = DMEK). For DMEK, pathological corneal endothelial cells were mechanically peeled off together with the basal membrane, i.e., the Descemet's membrane, and immersed in a corneal preservation solution Optisol-GS (Bausch & Lomb). Collagenase treatment was then applied to enzymatically collect the corneal endothelial cells, and the cells were cultured in a SB203580 + SB431542 + 3T3 conditioned medium. As for cultured corneal endothelial cells from a Fuchs' endothelial corneal dystrophy patient, an SV40 large T antigen and an hTERT gene were amplified by PCR and introduced into a lentiviral vector (pLenti6.3V5-TOPO; Life Technologies Inc). The lentiviral vector was then used to infect 293T cells (RCB2202; Riken Bioresource Center, Ibaraki, Japan) with a transfection reagent (Fugene HD; Promega Corp., Madison, WI) and three types of helper plasmids (pLP1, pLP2, pLP/VSVG; Life Technologies Inc.). Culture supernatant comprising viruses was collected after 48 hours from the infection. 5 µg/ml of polybrene was used to be added to a culture solution of cultured corneal endothelial cells from a Fuchs' endothelial corneal dystrophy patient, and an SV40 large T antigen and an hTERT gene were introduced. Images of immortalized corneal endothelial cell line (iFECD) from Fuchs' endothelial corneal dystrophy patients from a phase difference microscope were studied. Cultured corneal endothelial cells from a research cornea imported from the Seattle Eye Bank were immortalized by the same method to make an immortalized cell line of normal corneal endothelial cells (iHCEC). When images of the immortalized corneal endothelial cell line (iFECD) and the immortalized corneal endothelial cell line from a healthy donor (iHCEC) from a phase difference microscope are studied, both iHCEC and iFECD have a layer of polygonal form as normal corneal endothelial cells. IHCEC and iFECD were maintained and cultured in Dulbecco's Modified Eagle Medium (DMEM) + 10% fetal bovine serum (FBS).

### (Example 2: Screening using immortalized Fuchs' endothelial corneal dystrophy cells)

In this example, screening of a compound effective against a corneal endothelial disease was performed using the immortalized Fuchs' endothelial corneal dystrophy cells prepared in Example 1. In this example, TGF-β2 was used as a substance that induces cell damage to corneal endothelial cells.

### (Materials and Methods)

7 × 10³ immortalized Fuchs' endothelial corneal dystrophy cells were seeded on a 96-well plate and were cultured for 24 hours under the condition of 5% CO₂ at 37°C. Dulbecco's Modified Eagle Medium (DMEM) (nacalai tesque, 26252-94) + 10% FBS (Biological Industries/04-001-1A) + 1% penicillin-streptomycin (nacalai tesque, 26252-94) was used as the medium. Next, TGF-β2 (manufacturer: R&D Systems, Inc., distributor: Wako Pure Chemical Industries, Ltd./manufacturer codes 302-B2-002, 302-B2-010, distributor codes: 553-62881, 559-62883) (5 ng/mL) alone, or both TGF-β2 (5 ng/mL) and each agent of SCREEN-WELL® FDA Approved Drug Library V2, Japan version (10 µM) were added. After 28 hours under the condition of 5% CO₂ at 37°C, cell morphology was observed using a phase difference microscope. Caspase 3/7 activity was then measured using Caspase-Glo 3/7 Assay (Promega, #G8091). DMEM + 2% FBS + 1% P/S was used as the medium. Caspase 3/7 activity and cell viability in this example were measured using Veritas™ Microplate Luminometer. Such a device enables many samples to be simultaneously measured.

The control group was supplemented with a solvent of each reagent, i.e., DMSO (Dimethyl Sulfoxide, Sterile-filtered) (nacalai tesque, 13408-64). Further, Z-VD-FMK (isomer mixture) (Wako Pure Chemical Industries, Ltd./262-02061) (10 µM), which is a caspase inhibitor, was used as the positive control. In the primary screening, measurement of Caspase 3/7 activity was carried out with n = 2. Secondary screening was carried out with n = 5 for the top ranked agents. Caspase activity was measured after being cultured in Dulbecco's Modified Eagle Medium (DMEM) + 2% fetal bovine serum (FBS) + 1% penicillin/streptomycin (P/S) for 28 hours. The outline of the screening in the Example is shown in Figure **2****.**

### (Results)

### (Primary screening)

Figure **3** shows typical results of Caspase 3/7 activities measured by Caspase-Glo® 3/7 Assay in a primary screening. Measurement was carried out with n = 2 as the primary screening and a TGF-β2 (5 ng/mL) supplemented group was set as 100%. The horizontal axis of the graph in Figure **3****,** from the left, shows control group, TGF-β2 (5 ng/mL) supplemented group, TGF-β2 (5 ng/mL) + Z-VD-FMK supplemented group (10 µM) and TGF-β2 (5 ng/mL) + each agent of SCREEN-WELL® FDA Approved Drug Library V2, Japan version supplemented group (10 µM). (1) and (2) of the control group and the like shown in Figure **3** show the results of placement at the left end and right end of 96-well plate, respectively. (1) and (2) of the control group, TGF-β2(5ng/mL) supplemented group, TGF-β2 (5 ng/mL) + Z-VD-FMK supplemented group (10 µM) are n = 5 in total.

### (Confirmation of the precision of the assay system)

Typical indicators that are normally used in screening are shown below.
- CV value (coefficient of variation); CV (%) = standard deviation (SD)/average (Av) is an indicator showing the degree of dispersion. CV value of 10% or within is typically required in screening.
- S/B ratio (Signal/Background ratio); S/B = Av 100%/Av 0% is a ratio of signal intensities before and after a reaction. The larger the S/B ratio is, the larger the read width becomes, which makes it easier to determine the presence of activity. S/B ratio is preferably 2 or more.
- S/N ratio (Signal/Noise ratio); S/N = (Av 100% - Av 0%)/SD 0% is a ratio of the size of a signal to the dispersion of a base.
- Z'-factor; Z'-factor = 1-(3 × SD 100% + 3 × SD 0%) / (Av 100% - Av 0%) is a number that becomes a criterion for the quality of the assay system, which is calculated from data dispersion and signal intensity, which is the most important indicator for expressing precision of the assay system, wherein a system would generally pass as a system for screening if the Z' value is 0.5 or higher.

Figure **4** shows the result of calculating each indicator of when the control is set as 0% and the post-TGF-β2 reaction is set as 100% and when the post-TGF-β2 reaction is set as 100% and the positive control post-TGF-β2 + Z-VD-FMK reaction is set as 0%. As shown, the values that are generally required as a screening system are satisfied.

### (Secondary screening: Measurement of cell viability)

Cell viability is calculated as an indicator of toxicity with n = 2 in CellTiter-Glo® Luminescent Cell Viability Assay (Promega catalogue number: G7570). The cell viability was measured after culturing the cells in Dulbecco's Modified Eagle Medium (DMEM) + 2% fetal bovine serum (FBS) + 1% penicillin/streptomycin (P/S) for 28 hours. The cell viability was measured in the absence of TGF-β. Typical results of measurements of cell viability in a secondary screening are shown in Figure **5****.** When the cell viability was below 90%, the cells were removed from ranking.

### (Secondary screening: Measurement of Caspase 3/7 activity)

Evaluation of Caspase 3/7 activity was carried out with n = 5 in the secondary screening. Typical results are shown in Figure **6****.**

### (Evaluation of cell viability and Caspase 3/7 activity)

Figure **7** and Figure **8** show the agent name of each agent of SCREEN-WELL® FDA Approved Drug Library V2, Japan version, results of the secondary screening of Caspase 3/7 activity under TGF-β2 stimulation, results of cell viability, and values obtained by dividing Caspase 3/7 activity by the cell viability ((Caspase 3/7 activity)/(cell viability) (%)). It is shown that the lower the value obtained by dividing a value of Caspase 3/7 activity by a value of cell viability (Caspase 3/7 activity)/(cell viability) (%) is, the more useful the agent is in the secondary screening. It is expected that the agents shown in Figure **7** and Figure **8** exhibit Caspase 3/7 activity/cell viability (%) of 80% or less, have inhibiting activity against TGF-β2, are less toxic, and are effective against a disease in a corneal endothelium or the like. Figure **7** shows the agents of which value of Caspase 3/7 activity/cell viability (%) was ranked from the 1st to the 25th, and Figure **8** shows the agents of which value of Caspase 3/7 activity/cell viability (%) was ranked from the 26th to the 47th.

### (Example 3: Screening using immortalized human corneal endothelial cells)

In this example, screening of a compound effective against a corneal endothelial disease was performed using the immortalized human corneal endothelial cells prepared in Example 1. In this example, MG-132, which is known as a substance that induces endoplasmic reticulum (ER) associated stress, was used as a substance that induces cell damage.

### (Materials and Methods)

7 × 10³ immortalized human corneal endothelial cells (iHCEC) were seeded on a 96-well plate and were cultured for 24 hours under the condition of 5% CO₂ at 37°C. Dulbecco's Modified Eagle Medium (DMEM) (nacalai tesque, 26252-94) + 10% FBS (Biological Industries/04-001-1A) + 1% penicillin-streptomycin (nacalai tesque, 26252-94) was used as the medium. Next, MG-132 (SIGMA, M7449) (0.1 µM) alone, or both MG-132 (0.1 µM) and each agent of the top 40 types that exhibited the value of (Caspase 3/7 activity)/(cell viability) (%) of 80% or less in Example 2 (10 µM) were added. After 18 hours under the condition of 5% CO₂ at 37°C, cell morphology was observed using a phase difference microscope. Caspase 3/7 activity was then measured using Caspase-Glo® 3/7 Assay (Promega, #G8091). In the same manner as found in a patient with Fuchs' endothelial corneal dystrophy, MG-132 has action of increasing accumulation of unfolded proteins in cells and induces cell death via endoplasmic reticulum stress. DMEM + 2% FBS + 1% P/S was used as the medium. Furthermore, the control group was supplemented with a solvent of each reagent, i.e., DMSO (Dimethyl Sulfoxide, Sterile-filtered) (nacalai tesque, 13408-64). Further, Z-VD-FMK (isomer mixture) (Wako Pure Chemical Industries, Ltd./262-02061) (10 µM), which is a caspase inhibitor, was used as the positive control. The outline of the screening of this example is shown in Figure **9****.** Caspase 3/7 activity and cell viability were measured by the same procedure as Example 2 (n = 2 for each of them) . Caspase activity and cell viability were measured after culturing the cells in Dulbecco's Modified Eagle Medium (DMEM) + 2% fetal bovine serum (FBS) + 1% penicillin/streptomycin (P/S) for 18 hours.

### (Results)

Figure **10** shows the name of each agent, results of screening of Caspase 3/7 activity in MG-132 (0.1 µM) stimulation, results of cell viability in MG-132 (0.1 µM) stimulation, and values obtained by dividing a value of Caspase 3/7 activity by a value of the cell viability ((Caspase 3/7 activity)/(cell viability) (%)). It is expected that the agents shown in Figure **10** exhibit Caspase 3/7 activity/cell viability (%) of 80% or less, have inhibiting activity against TGF-β2, are less toxic, and are effective against a corneal endothelial disease or the like.

As disclosed above, the present invention is exemplified by the use of its preferred embodiments. However, it is understood that the scope of the present invention should be interpreted solely based on the Claims. It is also understood that any patent, any patent application, and any references cited herein should be incorporated herein by reference in the same manner as the contents are specifically described herein.

The present application claims priority to Japanese Patent Application No. 2017-118625 (filed on June 16, 2017). The entire content thereof is incorporated herein by reference.

### [Industrial Applicability]

The present invention provides a method for screening a compound effective against a corneal endothelial disease using a Fuchs' endothelial corneal dystrophy model system and provides a technique available to industries (pharmaceutical or the like) involved in corneal endothelial disease treatment.

## Claims

1. A method for screening a compound effective against a corneal endothelial condition, disorder, or disease, the method comprising:
(a) a step of contacting a candidate compound with immortalized Fuchs' endothelial corneal dystrophy cells in the presence of a transforming growth factor (TGF)-β to evaluate inhibiting activity of the candidate compound against the TGF-β and selecting a compound having the inhibiting activity;
(b) a step of evaluating toxicity of the compound selected in the step (a) to the cells;
(c) a step of evaluating the inhibiting activity of the compound selected in the step (a) against the TGF-β, if necessary; and
(d) a step of selecting a compound evaluated to be less toxic to the cells in the step (b) and evaluated to have the inhibiting activity against the TGF-β in the step (a) or the step (c).

2. The method of claim 1, wherein the inhibiting activity against the TGF-β is activity of inhibiting apoptosis of the cells due to the TGF-β signal.

3. The method of claim 1 or 2, wherein the steps (a) and (c) comprise measuring caspase activity, wherein the inhibiting activity against the TGF-β is caspase activity which is substantially decreased compared to a candidate compound non-contacted group.

4. The method of any one of claims 1 to 3, wherein the number of procedures of evaluation of the inhibiting activity against the TGF-β in the step (c) is larger than the number of procedures of evaluation of the inhibiting activity against the TGF-β in the step (a).

5. The method of claim 4, wherein the number of procedures of evaluation of the inhibiting activity against the TGF-β in the step (c) is n = 4 to 6 and the number of procedures of evaluation of the inhibiting activity against the TGF-β in the step (a) is n = 1 to 3.

6. The method of any one of claims 3 to 5, wherein the caspase activity is Caspase 3/7 activity.

7. The method of claim 6, wherein a compound exhibiting Caspase 3/7 activity that is about 75% or less compared to a candidate compound non-contacted group is selected as a compound having the inhibiting activity against the TGF-β in the step (a).

8. The method of any one of claims 1 to 7, wherein the step (b) comprises measuring cell viability, wherein the toxicity is evaluated to be low when the cell viability is not substantially reduced.

9. The method of claim 8, wherein a compound exhibiting cell viability which is not substantially reduced and Caspase 3/7 activity which is substantially decreased compared to a candidate compound non-contacted group is selected in the step (d).

10. The method of claim 9, wherein a compound exhibiting about 90% or more of the cell viability and exhibiting a ratio of Caspase 3/7 activity (%) to the cell viability (%) that is about 0.8 or less is selected in the step (d).

11. The method of any one of claims 1 to 10, wherein the corneal endothelial condition, disorder, or disease is Fuchs' endothelial corneal dystrophy.

12. The method of any one of claims 1 to 11, further comprising:
(e) a step of contacting a compound selected in the step (d) with immortalized human corneal endothelial cells in the presence of an endoplasmic reticulum associated stress inducing substance to evaluate inhibiting activity of a compound selected in the step (d) against the endoplasmic reticulum associated stress inducing substance;
(f) a step of evaluating toxicity of a compound selected in the step (d) against the immortalized human corneal endothelial cells; and
(g) a step of selecting a compound which was evaluated to be less toxic to the immortalized human corneal endothelial cells in step (f) and evaluated to have the inhibiting activity against the endoplasmic reticulum associated stress inducing substance in step (e).

13. The method of claim 12, wherein the inhibiting activity against the endoplasmic reticulum associated stress inducing substance is activity of inhibiting apoptosis of the immortalized human corneal endothelial cells due to the endoplasmic reticulum associated stress inducing substance.

14. The method of claim 12 or 13, wherein the step (e) comprises measuring caspase activity, wherein the inhibiting activity against the endoplasmic reticulum associated stress inducing substance is caspase activity which is substantially decreased compared to a candidate compound non-contacted group.

15. The method of claim 14, wherein the caspase activity is Caspase activity 3/7.

16. The method of claim 15, wherein a compound exhibiting Caspase 3/7 activity that is about 75% or less compared to a candidate compound non-contacted group is selected as a compound having the inhibiting activity against the endoplasmic reticulum associated stress inducing substance in the step (g).

17. The method of any one of claims 12 to 16, wherein the step (f) comprises measuring cell viability, wherein the toxicity is evaluated to be low when the cell viability is not substantially reduced.

18. The method of claim 17, wherein a compound exhibiting cell viability which is not substantially reduced and Caspase 3/7 activity which is substantially decreased compared to a candidate compound non-contacted group is selected in the step (g).

19. The method of claim 18, wherein a compound exhibiting about 90% or more of the cell viability and exhibiting a ratio of Caspase 3/7 activity (%) to the cell viability (%) that is about 0.8 or less is selected in in the step (g).

20. The method of any one of claims 12 to 19, wherein the corneal endothelial condition, disorder, or disease is Fuchs' endothelial corneal dystrophy.

21. The method of any one of claims 12 to 20, wherein the endoplasmic reticulum associated stress inducing substance is selected from the group consisting of MG-132, thapsigargin, and tunicamycin.

22. The method of claim 21, wherein the endoplasmic reticulum associated stress inducing substance is MG-132.

23. A method for screening a compound effective against a corneal endothelial condition, disorder, or disease, the method comprising:
(a) a step of contacting a candidate compound with immortalized Fuchs' endothelial corneal dystrophy cells in the presence of a transforming growth factor (TGF)-β to evaluate caspase activity of the candidate compound and selecting a compound with substantially decreased caspase activity compared to a candidate compound non-contacted group;
(b) a step of evaluating toxicity of the compound selected in the step (a) to the immortalized Fuchs' endothelial corneal dystrophy cells;
(c) a step of evaluating the caspase activity of the compound selected in the step (a);
(d) a step of selecting a compound evaluated to be less toxic to the immortalized Fuchs' endothelial corneal dystrophy cells in the step (b) and evaluated to have caspase activity which is substantially decreased in the step (c);
(e) a step of contacting a compound selected in the step (d) with immortalized human corneal endothelial cells in the presence of MG-132 to evaluate caspase activity of a candidate compound of a compound selected in the step (d);
(f) a step of evaluating toxicity of the immortalized human corneal endothelial cells of a compound selected in the step (e); and
(g) a step of selecting a compound which was evaluated to be less toxic to the immortalized human corneal endothelial cells in the step (f) and evaluated to have caspase activity which is substantially decreased in the step (e).

24. A kit for screening a compound effective against a corneal endothelial condition, disorder, or disease using the method of any one of claims 1 to 11, the kit comprising:
a TGF-β;
immortalized Fuchs' endothelial corneal dystrophy cells;
a reagent or device for measuring the inhibiting activity against the TGF-β; and
a reagent or device for measuring cell toxicity.

25. The kit of claim 24, wherein the TGF-β is TGF-β2.

26. The kit of claim 24 or 25, wherein the immortalized Fuchs' endothelial corneal dystrophy cells are from corneal endothelial cells from a Fuchs' endothelial corneal dystrophy patient, to which an SV40 large T antigen and an hTERT gene were introduced.

27. The kit of claim 26 wherein the immortalized Fuchs' endothelial corneal dystrophy cells are from immortalized cells from a Fuchs' endothelial corneal dystrophy patient which were cultured in a medium comprising 4-[4-(1,3-benzodioxole-5-yl)-5-(2-pyridinyl)-1H-imidazole-2-yl]benzamide and 4-[4-(4-fluorophenyl)-2-(4-methylsulfinylphenyl)-1H-imidazole-5-yl]pyridine).

28. The kit of any one of claims 24 to 27, wherein the reagent or device for measuring the inhibiting activity against the TGF-β is a reagent or device for measuring caspase activity.

29. The kit of any one of claims 24 to 28, wherein the caspase activity is Caspase 3/7 activity.

30. The kit of any one of claims 24 to 29, wherein the reagent or device for measuring the cell toxicity is a reagent or device for measuring cell viability.

31. The kit of any one of claims 24 to 30, wherein the reagent or device for measuring the inhibiting activity against the TGF-β comprises a luminescence reagent.

32. The kit of any one of claims 24 to 31, wherein the reagent or device for measuring the cell toxicity comprises a luminescence reagent.

33. A method for screening a compound effective against a corneal endothelial condition, disorder, or disease, the method comprising:
(a) a step of contacting a candidate compound with immortalized human corneal endothelial cells in the presence of an endoplasmic reticulum associated stress inducing substance to evaluate inhibiting activity of the candidate compound against the endoplasmic reticulum associated stress inducing substance and selecting a compound having the inhibiting activity;
(b) a step of evaluating toxicity of the compound selected in the step (a) to the cells;
(c) a step of evaluating the inhibiting activity of the compound selected in the step (a) against the endoplasmic reticulum associated stress inducing substance, if necessary; and
(d) a step of selecting a compound evaluated to be less toxic to the cells in the step (b) and evaluated to have the inhibiting activity against the endoplasmic reticulum associated stress inducing substance in the step (a) or the step (c).

34. The method of claim 33, wherein the inhibiting activity against the endoplasmic reticulum associated stress inducing substance is activity of inhibiting apoptosis of the cells due to the endoplasmic reticulum associated stress inducing substance.

35. The method of claim 33 or 34, wherein the steps (a) and (c) comprise measuring caspase activity, wherein the inhibiting activity against the endoplasmic reticulum associated stress inducing substance is caspase activity which is substantially decreased compared to a candidate compound non-contacted group.

36. The method of any one of claims 33 to 35, wherein the number of procedures of evaluation of the inhibiting activity against the endoplasmic reticulum associated stress inducing substance in the step (c) is larger than the number of procedures of evaluation of the inhibiting activity against the endoplasmic reticulum associated stress inducing substance in the step (a).

37. The method of claim 36, wherein the number of procedures of evaluation of the inhibiting activity against the endoplasmic reticulum associated stress inducing substance in the step (c) is n = 4 to 6 and the number of procedures of evaluation of the inhibiting activity against the endoplasmic reticulum associated stress inducing substance in the step (a) is n = 1 to 3.

38. The method of any one of claims 35 to 37, wherein the caspase activity is Caspase 3/7 activity.

39. The method of claim 38, wherein a compound exhibiting Caspase 3/7 activity that is about 75% or less compared to a candidate compound non-contacted group is selected as a compound having the inhibiting activity against the endoplasmic reticulum associated stress inducing substance in the step (a).

40. The method of any one of claims 33 to 39, wherein the step (b) comprises measuring cell viability, wherein the toxicity is evaluated to be low when the cell viability is not substantially reduced.

41. The method of claim 40, wherein a compound exhibiting cell viability which is not substantially reduced and Caspase 3/7 activity which is substantially decreased compared to a candidate compound non-contacted group is selected in the step (d).

42. The method of claim 41, wherein a compound exhibiting 90% or more of the cell viability and exhibiting a ratio of Caspase 3/7 activity (%) to the cell viability (%) that is about 0.8 or less is selected in the step (d).

43. The method of any one of claims 33 to 42, wherein the corneal endothelial condition, disorder, or disease is Fuchs' endothelial corneal dystrophy.

44. The method of any one of claims 33 to 43, wherein the endoplasmic reticulum associated stress inducing substance is selected from the group consisting of MG-132, thapsigargin, and tunicamycin.

45. The method of claim 44, wherein the endoplasmic reticulum associated stress inducing substance is MG-132.

46. A kit for screening a compound effective against a corneal endothelial condition, disorder, or disease using the method of any one of claims 33 to 45, the kit comprising:
an endoplasmic reticulum associated stress inducing substance;
immortalized human corneal endothelial cells;
a reagent or device for measuring the inhibiting activity against the endoplasmic reticulum associated stress inducing substance; and
a reagent or device for measuring cell toxicity.

47. The kit of claim 46, wherein the immortalized human corneal endothelial cells are from normal corneal endothelial cells, to which an SV40 large T antigen and an hTERT gene were introduced.

48. The kit of claim 46 or 47 wherein the immortalized human corneal endothelial cells are from normal immortalized cells which were cultured in a medium comprising 4-[4-(1,3-benzodioxole-5-yl)-5-(2-pyridinyl)-1H-imidazole-2-yl]benzamide and 4-[4-(4-fluorophenyl)-2-(4-methylsulfinylphenyl)-1H-imidazole-5-yl]pyridine).

49. The kit of any one of claims 46 to 48, wherein the reagent for measuring the inhibiting activity against the TGF-β is a reagent for measuring caspase activity.

50. The kit of any one of claims 46 to 49, wherein the caspase activity is Caspase 3/7 activity.

51. The kit of any one of claims 46 to 50, wherein the reagent for measuring the cell toxicity is a reagent for measuring cell viability.

52. The kit of any one of claims 46 to 51, wherein the endoplasmic reticulum associated stress inducing substance is selected from the group consisting of MG-132, thapsigargin, and tunicamycin.

53. The kit of any one of claims 46 to 52, wherein the endoplasmic reticulum associated stress inducing substance is MG-132.

54. The kit of any one of claims 46 to 53, wherein the reagent or device for measuring the inhibiting activity against the endoplasmic reticulum associated stress inducing substance comprises a luminescence reagent.

55. The kit of any one of claims 46 to 54, wherein the reagent or device for measuring the cell toxicity comprises a luminescence reagent.
